# EUROPÄISCHE PATENTSCHRIFT

(11) **EP 3 750 580 B1**
(45) Veröffentlichungstag und Bekanntmachung des Hinweises auf die Patenterteilung: **24.12.2025**
(21) Anmeldenummer: 19180053.1
(22) Anmeldetag: 13.06.2019
(51) Int. Cl.: A61M 5/31, A61M 5/32, A61M 5/34

(54) **MIT EINEM FLUID VORFÜLLBARER ODER VORGEFÜLLTER BEHÄLTER SOWIE KANÜLENBAUGRUPPE UND VERSCHLUSSSYSTEM FÜR EINEN MIT EINEM FLUID VORFÜLLBAREN ODER VORGEFÜLLTEN BEHÄLTER**
CONTAINER WHICH IS PREFILLED OR CAN BE PREFILLED WITH A FLUID AND CANNULA ASSEMBLY AND CLOSURE SYSTEM FOR SAME
RÉCIPIENT POUVANT ÊTRE PRÉ-REMPLI OU PRÉ-REMPLI D'UN FLUIDE AINSI QUE MODULE DE CANULES ET SYSTÈME DE FERMETURE POUR UN RÉCIPIENT POUVANT ÊTRE PRÉ-REMPLI OU PRÉ-REMPLI D'UN FLUIDE

(43) Veröffentlichungstag der Anmeldung: 16.12.2020
(73) Patentinhaber: SCHOTT Pharma Schweiz AG, 9000 St. Gallen (CH)
(72) Erfinder: VAN GINNEKEN, Tom, 9404 Rorschacherberg (CH)
(74) Vertreter: Blumbach · Zinngrebe Patentanwälte PartG mbB

(56) Entgegenhaltungen:
- EP-A2- 1 364 670
- CH-A5- 583 567
- GB-A- 377 891
- US-A- 2 034 294
- US-B1- 6 488 666

## Beschreibung

Die vorliegende Erfindung betrifft einen mit einem Fluid, insbesondere mit einem pharmazeutischen oder kosmetischen Präparat, vorfüllbaren oder vorgefüllten Behälter, insbesondere mit einem pharmazeutischen oder kosmetischen Präparat, vorfüllbaren oder vorgefüllten Behälter, wobei der Behälter eine Spritze oder Karpule, einen Spritzenkörper oder eine Injektionsvorrichtung umfassen kann.

Das Fluid, insbesondere das pharmazeutische oder kosmetische Präparat, kann in dem Behälter gelagert und/oder mittels des Behälters injiziert oder appliziert werden. Insgesamt steht bei einer vorgefüllten Spritze zwar zunächst das Injizieren oder Einspritzen als Applizieren im Vordergrund, aber mit dem Gegenstand der vorliegenden Erfindung kann auch Flüssigkeit entnommen werden. Derartige Systeme können beispielsweise auch zum Dosieren beispielsweise für eine Infusion eingesetzt werden. Ferner kann zwar aber muss das Applizieren kein Eindringen in den menschlichen oder tierischen Körper umfassen, sondern kann auch das Aufbringen, wie beispielsweise das Auftropfen eines Anästhetikums auf eine Körperregion eines Patienten umfassen, wie dies beispielsweise zur Lokalanästhesie in der Oralchirurgie vor dem Einstechen und Injizieren eines Anästhetikums häufig vorgenommen wird.

Ferner umfasst die Bezeichnung medizinisches Präparat im Rahmen der vorliegenden Offenbarung auch veterinärmedizinische Präparate und deren entsprechende Anwendungen.

Hierbei kommt der sachgerechten Lagerung sowie der Applikation dieser Präparate in der Regel erhebliche Bedeutung zu, insbesondere ist auch die Sterilität während der gesamten Lagerungsdauer von erheblicher Bedeutung.

Die im Rahmen der vorliegenden Offenbarung diskutierten Behälter für kosmetische oder pharmazeutische Präparate, beispielsweise Spritzen oder Karpulen, leisten auch einen wesentlichen Beitrag im Rahmen der Verabreichung dieser Präparate. Diese im allgemeinen zylindrisch geformten Behälter umfassen typischerweise eine Kammer zum Aufnehmen des Präparates, wobei die Kammer von einem proximalen und einem distalen Ende begrenzt wird.

Als proximales Ende wird im Rahmen der vorliegenden Offenbarung jeweils dasjenige Ende verstanden, in welches beispielsweise ein Stopfen oder Kolbe eingebracht wird, während als distales Ende dasjenige Ende verstanden wird, an welchem beispielsweise eine Kanüle mit deren Kanülenbaugruppe anbringbar ist. Im Rahmen einer verbreiteten Bezeichnung in der einschlägigen Fachliteratur wird auch das bei der üblichen Handhabung dem Benutzer zugewandte Ende als proximales Ende und das dem Benutzer ab- und bei der Applikation kosmetischer und/oder medizinischer Präparate dem Patienten zugewandte Ende als distales Ende bezeichnet.

Soweit diese Begriffe "proximal" und "distal" bei verschiedenen Baugruppen verwendet werden, sind hierbei jeweils die vorstehend erläuterten geometrischen Verhältnisse in gleicher Weise für die jeweilige Baugruppe offenbart.

Während einer typischen Applikation des pharmazeutischen oder kosmetischen Präparats wird somit das Ende proximale einem Benutzer zu und das distale dem Benutzer abgewandt sein.

Ein Kolben kann insbesondere in das proximale Ende eingesetzt werden, um in einer axialen, insbesondere gleitenden Bewegung das Präparat in distale Richtung und durch einen Durchgangskanal am distalen Ende des Behälters zu führen oder in einer umgekehrten Bewegung ein Präparat oder auch eine Körperflüssigkeit eines Patienten durch diese Öffnung in den Behälter, insbesondere in die Spritze einziehen. Das den Durchgangskanal umgebende Endstück des Behälters ist dabei häufig zumindest bereichsweise als konisch zulaufende Spitze oder Konus ausgebildet.

Zum Injizieren oder Applizieren eines Präparates kann auf diesen Konus eine Kanüle aufgesetzt werden, wie es beispielsweise in der Druckschrift GB 377,891 beschrieben ist. Das hier beschriebene Aufsetzen auf den Konus ermöglicht ein einfaches Austauschen der Kanüle. Die US 2,034,294 beschreibt ein ähnliches Prinzip, um eine Kanüle mit einem Konus mit einer Spritze verbinden zu können.

Das Patent US 6 488 666 B1 beschreibt eine Vorrichtung, mit der ein Einstechen gebrauchter Kanülen verhindert werden soll. Hierzu wird die Kanüle nach dem Gebrauch in eine Hülse aufgenommen und durch eine Verriegelungseinheit fixiert. Die Kanüle wird dabei zwischen der Hülse und der elastischen Verriegelungseinheit eingeschlossen.

Eine weitere Ausführungsform zum Verbinden einer Kanüle mit einer Spritze beschreibt die Druckschrift WO 02/087671 A1. Um Gefahren vorzubeugen, welche mit einer auf einer Spritze fest montierten Kanüle einhergehen, etwa mögliche Übertragung von Krankheiten durch Kontakt mit einer gebrauchten Kanüle, wird ein System vorgeschlagen, bei welchem die Kanüle in die Kammer des Behälters nach dem Applizieren eines Präparates einziehbar ist. Dabei wird die gesamte Kanüle durch Zurückziehen des Kolbens in die Kammer gezogen.

Für ein schnelleres und einfacheres Applizieren pharmazeutischer Präparate werden heutzutage immer häufiger bereits vorgefüllte Spritzen oder Karpulen eingesetzt. Hierdurch entfällt das Überführen des Wirkstoffes von einem ersten Vorratsbehälter in die Kammer des Behälters vor der Verabreichung, was zum einen die Zeit zur Applikation verkürzt, zum anderen aber auch Verluste beim Überführen der Präparate vermeiden hilft. Dies ist insbesondere bei sehr hochwertigen Medikamenten von Vorteil. Weiterhin kann auch die Gefahr von Kontaminationen der Wirkstoffe und von Infektionen vermindert werden.

Hierzu sind im medizinischen Bereich Behälter aus Kunststoff bekannt, welche mit einem Verschluss versehen sind, und wie sie beispielsweise in der Druckschrift DE 10 2014 007 604 A1 genannt sind. Vorgefüllte Behälter können mit dem Verschluss abgedichtet werden, wobei der Verschluss dann zum Applizieren des Präparates geöffnet und stattdessen eine Kanüle angebracht werden kann.

Ein weiterer Behälter ist beispielsweise in der DE 10 2014 211 018 A1 beschrieben. Der in dieser Druckschrift beschriebene Behälter ist aus Glas gefertigt, um die inerten Eigenschaften des Glases in Bezug auf teure, hochwirksame Medikamente in vorteilhafter Weise nutzen zu können. Eine Öffnung des Behälters aus Glas wird mit einem Anschlusskörper aus Kunststoff verschlossen, welcher die Behälterkammer verschließt und den Austritt eines in diesen gefüllten Präparates verhindert. Zur späteren Applikation des in den Behälter eingefüllten Präparates wird der Anschlusskörper geöffnet und der Behälter an dieser Stelle mit einer Kanüle verbunden, mittels welcher nachfolgend das Präparat verabreicht werden kann.

Zwar können auf diese Weise vorgefüllte Spritzen hergestellt werden, das Applizieren des Präparates ist jedoch immer noch mit einem gewissen Aufwand verbunden, da die Behälterkammer vor dem Applizieren jeweils zu öffnen ist. Zudem muss eine passende Kanüle gefunden und ebenfalls geöffnet und sodann mit der vorgefüllten Spritze verbunden werden. Neben dem erhöhten Zeitaufwand birgt auch dieser Wechsel ein gewisses Risiko einer Kontamination, falls beispielsweise die geöffnete Kanüle in Kontakt mit einer unerwünschten Substanz tritt, bevor diese an der Spritze montiert ist.

Es sind ferner vorgefüllte Einwegspritzen mit gebrauchsfertiger Medikamentenfüllung bekannt. Es wird beispielsweise in der Druckschrift DE 27 17 830 A1 eine vorgefüllte Einwegspritze beschrieben, bei welcher bereits eine Kanüle vorhanden ist.

Auch die Druckschrift EP 1 364 670 A2 beschreibt eine Einmalspritze mit einer Kanüle in einem Nadelkanal der Spritze.

Die Druckschrift CH 583 567 A5 beschreibt einen Infusionssatz für einen Behälter mit einem Flansch, welcher eine Kanüle aufnimmt und haltert. Der Flansch ist nicht direkt mit dem Hals des Behälters verbunden, sondern mit einem Zwischenstück mit einer Membran, welche den Behälterhals verschließt. Die Druckschrift US 6 488 666 B1 beschreibt eine Vorrichtung zur Verhinderung von Stichen einer gebrauchten Nadel.

Bei derartigen Einwegspritzen wird zunächst die Kanüle mit dem Behälterkörper verbunden, und sodann erfolgt das Befüllen der Spritze mit dem pharmazeutischen Präparat. Bei Behältern aus Kunststoff wird die Kanüle häufig bereits bei der Erzeugung des Behälters mit diesem befestigt, welches typischerweise im Spritzgussverfahren erfolgt, d.h. die Kanüle wird zum Beispiel im Spritzguss umspritzt und damit stoffschlüssig mit dem Behälter verbunden. Dies ist jedoch ungünstig, da für jede Geometrie von Kanüle oder Behälter entsprechende Spritzwerkzeuge bereitzustellen sind.

Dies bedeutet zudem, dass während des Herstellprozesses der Behälterkörper zusammen mit der Kanüle bewegt werden muss, welches eine besondere Sorgfalt bei deren jeweiliger Handhabung erfordert.

Ein weiterer wesentlicher Nachteil liegt darin, dass Kanülen mit relativ kleinem Durchmesser regelmäßig nicht im Spritzgussverfahren mit dem Behälterkörper verbunden werden können. Eine gängige Größenbezeichnung der Kanüle in Bezug auf deren Außendurchmesser ist die Größenangabe in Gauge (G). Kanülen mit einem Außendurchmesser von größer als 30 G, demnach Kanülen mit einer Größe von 31 G, 32 G, 33 G oder 34 G, können regelmäßig nicht im Spritzgussverfahren hergestellt werden. Demnach können keine vorgefüllten Einwegspritzen mit gebrauchsfertiger Medikamentenfüllung mit derartig dünnen Kanülen, insbesondere mit einer Größe zwischen 31 G bis 34 G, zur Verfügung gestellt werden. Dies ist insofern ungünstig, als dass beispielsweise bei dem Injizieren eines pharmazeutischen Präparates in den menschlichen Körper das Schmerzempfinden bei kleinerem Außendurchmesser der Kanüle abnimmt, weshalb hier möglichst Kanülen mit geringem Außendurchmesser eingesetzt werden.

Zudem wird die Variantenvielfalt, die sich beispielsweise in Bezug auf die Größe der Kammer für das Präparat in Verbindung mit unterschiedlichen Abmessungen der Kanüle ergeben kann, rasch sehr groß und kann zu einer hohen Anzahl an Werkzeugen zur Herstellung und einer aufwendigen Lagerhaltung führen.

Häufig wird die Kanüle auch in den Kanal der Spritze geklebt, welches allgemein als nachteilig angesehen wird, da hierbei ein zusätzliches Material zum Einsatz kommt, das, insbesondere über einen längeren Zeitraum wie beispielsweise von mehr als einem Jahr bis zu mehr als 3 Jahren gesehen, bei dem eingefüllten Präparat zu einer unerwünschten Kontamination führen kann. Als nachteilig wird hier auch eine mögliche Kontamination des pharmazeutischen Präparates durch Ausgasungen von oder aus dem verwendeten Klebstoff angesehen. Diese können beispielsweise ein eingefülltes Medikament kontaminieren und schädigen. Da eine derartige Kontamination für einen Anwender nicht sichtbar sein muss, sollte diese mit besonders hoher Sicherheit vermieden werden.

Wünschenswert wäre es demnach, einen vorfüllbaren Behälter für pharmazeutische Präparate zur Verfügung zu stellen, welcher mit einem Fluid, insbesondere mit einem pharmazeutischen oder kosmetischen Präparat, befüllt werden kann und bei welchem ein Applizieren oder Injizieren des Fluids aus dem beziehungsweise mittels des vorgefüllten Behälter möglich ist, ohne dass ein Demontieren eines Verschlusses und/oder ein Montieren einer Kanüle oder eines weiteren Bauelements erforderlich wird.

Dabei soll ein mit dem Fluid vorgefüllter Behälter nach dem Befüllen vorzugsweise auch eine verbesserte Lagerfähigkeit aufweisen, vorzugsweise von wenigstens einem Jahr, bevorzugt von wenigstens zwei Jahren, besonders bevorzugt von wenigstens drei Jahren oder sogar darüber hinaus. Der mit dem Fluid vorgefüllte Behälter soll, insbesondere in den Fällen, bei denen es sich bei dem Fluid um ein pharmazeutisches oder kosmetisches Präparat handelt, über diesen Zeitraum auch den geltenden Sterilitätsanforderungen, insbesondere denen im pharmazeutischen Bereich für die Lagerung von pharmazeutischen Präparaten geltenden, genügen.

Dabei soll möglichst auf vorhandene Geometrien der Behältnisse, insbesondere auf am Markt vorhandene Spritzen, Spritzenkörper, Karpulen oder Injektionsvorrichtungen, und Kanülen zurückgegriffen werden können.

Zusammenfassend liegt eine Aufgabe der Erfindung darin, einen in Bezug auf die Dimension des Behältnisses und die Kanüle flexiblen, vormontierten oder vormontierbaren, vorfüllbaren oder vorgefüllten Behälter zum Lagern und Applizieren eines Fluids, insbesondere eines pharmazeutischen oder kosmetischen Präparates, zur Verfügung zu stellen, welcher über einen längeren Zeitraum lagerbar ist.

Der vorfüllbarer oder vorgefüllte Behälter soll dabei vorteilhaft insbesondere mit sehr dünnen Kanülen ausgestattet werden können, insbesondere auch mit Kanülen einer Gauge-Größe zwischen 31 G bis 34 G.

Ferner soll ein vorgefüllter Behälter eine Lagerfähigkeit von wenigstens drei Jahren aufweisen, wobei die geltenden Sterilitätsanforderungen an die Lagerung des Fluids, insbesondere an die Lagerung von pharmazeutischen oder kosmetischen Präparaten, eingehalten werden sollen.

Dabei sollen ferner vorteilhaft modulartig am Markt erhältliche Behältnisse und Kanülen verwendet werden können.

Diese Aufgabe wird mit den Merkmalen des Anspruchs 1 gelöst, welcher insbesondere auch die vorstehend erwähnten vorteilhaften weiteren Wirkungen bereitstellt.

Gemäß der Erfindung wird ein vorfüllbarer oder vorgefüllter Behälter zum Lagern und/oder Applizieren eines Fluids, insbesondere eines pharmazeutischen oder kosmetischen Präparates, bereitgestellt umfassend
ein Behältnis,
   insbesondere eine Spritze, mit einem
Spritzenkörper, eine Karpule oder eine Injektionsvorrichtung, mit
   einer Kammer zur Aufnahme
   des Fluids

ferner umfassend
   eine Kanülenbaugruppe,
      wobei die Kanülenbaugruppe eine Kanüle
         und
      ein Dichtelement aufweist und
   die Kanülenbaugruppe an dem Behältnis anbringbar ist,
sowie umfassend eine Verschlusskappe,
wobei eine außenseitige Kanalöffnung der Kanüle in einen abdichtenden elastomeren Innenbereich der Verschlusskappe hineinragt und dieser die Kanalöffnung abdichtet,
wobei durch eine radiale und/oder axiale Kompression des Dichtelements sowie durch die Abdichtung der Kanalöffnung mittels der Verschlusskappe die Kammer fluiddicht verschlossen ist.

Die vorteilhaft ermöglichte Lagerzeit bzgl. Stabilität und Sterilität von mehr als drei Jahren wird insbesondere erzielt durch die axiale und/oder radiale Kompression des Dichtelements der Kanülenbaugruppe sowie die Abdichtung der Kanüle mittels der Verschlusskappe, wobei zumindest zwei Dichtflächen definiert werden: die Dichtfläche des Dichtelements zu einem vorderen Bereich oder distalen Ende des Behältnis, welcher vorzugsweise als Luer-Lock ausgebildet ist, und die Dichtung in der Verschlusskappe der Kanüle, welche außenseitige Kanalöffnung der Kanüle abdichtet.

Vorteilhaft ergibt sich hierbei die Verwendung eines vorgefüllten Behälters, wie dieser vorliegend offenbart wird für eine sterile Lagerung eines pharmazeutischen oder kosmetischen Präparats für eine Lagerdauer von mehr als einem, bevorzugt mehr als zwei, besonders bevorzugt mehr als drei Jahren.

Der Begriff vorgefüllter Behälter bezeichnet dabei einen befüllten Behälter, welcher nicht mehr durch den jeweiligen Anwender selbst befüllt werden muss, sondern beispielsweise durch den Hersteller des jeweiligen Präparates oder bei einer Weiterverarbeitung, beispielsweise mittels weiterer Füll- oder Wirkstoffen, durch den jeweiligen Weiterverarbeiter oder Befüller befüllt worden sein kann.

Aber auch der spätere Anwender kann den vorliegend offenbarten Behälter, beispielsweise in einem klinischen Umfeld selbst vorbefüllen, um diesen beispielsweise lagern zu können, insbesondere für einen längeren Zeitraum lagern zu können, sodass dieser insbesondere in zeitkritischen Situationen, wie diese beispielsweise in der Notfallmedizin auftreten können, sofort verwendbar ist. Hierdurch können auch Präparate für zeitkritische und/oder notfallmedinzinsche Einsatzzwecke länger lagerbar sein und deren regelmäßiger, durch das Ablaufdatum des jeweiligen Präparats bedingter nötiger Austausch deutlich vermindert werden.

Als sterile Lagerung wird hierbei eine Lagerung verstanden, welche die notwendigen Sterilitätsbedingungen über den jeweils angegebenen Zeitraum erfüllt. Für derartige Sterilitätsbedingungen wird typischerweise gefordert, dass ein wie vorliegend offenbarter vorbefüllter Behälter nach dessen Sterilisation in seinem Inneren eine Population von lebenden Individuen, beispielsweise von Keimen oder Mikroorganismen, von einem weniger als 10⁻⁶-Fachen aufweist, diese in deren Anzahl somit auf weniger als ein Millionsten reduziert wurden. Eine Lagerung, welche diese Bedingung über den jeweils angegebenen Zeitraum erfüllt, wird für diesen Zeitraum als sterile Lagerung betrachtet. Verfahren zur Sterilisierung sind dem Fachmann umfänglich bekannt und werden an späterer Stelle der vorliegenden Offenbarung nochmals diskutiert.

Die Kanülenbaugruppe und der Adapter dienen dazu, eine Kanüle mit einem Behältnis wie einer Spritze, einem Spritzenkörper, einer Karpule oder einer Injektionsvorrichtung fest und dauerhaft dicht zu verbinden und dabei die Öffnung an dem distalen Ende abzudichten, um einen vorfüllbaren Behälter zum Lagern und Applizieren eines Fluids, insbesondere eines pharmazeutischen oder kosmetischen Präparates, zu schaffen. Ein erfindungsgemäß vorfüllbarer oder vorgefüllter Behälter umfasst demnach zumindest ein derartiges Behältnis und eine an dem Behältnis befestigte Kanüle.

Das Behältnis kann zur Aufnahme des Fluids eine Kammer aufweisen, welche mit dem Fluid gefüllt sein oder gefüllt werden kann. Das Fluid kann ein pharmazeutisches oder kosmetisches Präparat umfassen. Es kann Flüssigkeiten, Gase, Feststoffe oder Gemische hieraus umfassen. Ein mit einem Fluid vorgefüllter Behälter kann über einen längeren Zeitraum gelagert werden, bevor mittels der Kanüle das Fluid appliziert werden kann.

Von Vorteil ist es, dass ein Demontieren eines Verschlusses und/oder ein Montieren einer Kanüle oder eines weiteren Bauelements vor dem Applizieren entbehrlich wird. Die Erfindung ermöglicht es, das Behältnis mit einem Fluid zu befüllen und davor oder danach fest und dauerhaft dicht eine Kanüle mit diesem Behältnis zu verbinden und dabei die Öffnung an dem distalen Ende abzudichten. Ein vorgefüllter Behälter kann dann über einen längeren Zeitraum gelagert und sodann "ready-to-use" zum Applizieren des Fluids, beispielsweise eines pharmazeutischen oder kosmetischen Präparates, zur Verfügung gestellt werden kann.

Der Herstellprozess ist dabei vergleichsweise einfach, da nicht bereits bei der Herstellung des Behältnisses die Kanüle mit montiert werden muss.

Erfindungsgemäß können am Markt erhältliche Behältnisse, insbesondere Spritzen, Spritzenkörper, Karpulen oder Injektionsvorrichtungen und Kanülen verwendet werden. Dies vereinfacht die Verfügbarkeit insofern, als dass diese Behältnisse und Kanülen auf vielfältige Weise miteinander kombiniert werden können und somit modulartig für den vorfüllbaren oder vorgefüllten Behälter ein sehr breites Spektrum in Bezug auf die Behältnisse und Kanülen abgebildet werden kann.

Dies führt vorteilhaft weiterhin dazu, dass die Anzahl an Vorrichtungen und Werkzeugen, beispielsweise die Anzahl an Spritzwerkzeugen, deutlich reduziert werden kann.

Somit können am Markt verfügbare Komponenten beliebig zu einer vorgefertigten Einheit kombiniert werden. Die Erfindung bietet damit den großen Vorteil einer Kostenreduktion, insbesondere in Bezug auf die Vorrichtungen und Werkzeuge, bei gleichzeitig höchster Format- und Kanülenvielfalt.

Zudem kann auf zusätzliche Komponenten wie beispielsweise Klebstoffe im Kontaktbereich mit dem Fluid verzichtet werden, was die Lagerfähigkeit verbessert, da eine unerwünschte Kontamination des Fluids mit Bestandteilen oder Reaktionsprodukten des Klebstoffes vermieden werden kann. Auch die Gefahr einer Kontamination des Fluids durch Ausgasungen aus dem Klebstoff kann damit vermieden werden, was insbesondere im Fall von pharmazeutischen oder kosmetischen Präparaten von großer Bedeutung ist.

Ein großer Vorteil der Erfindung liegt darin, dass die Verbindung von Kanüle und Behältnis dicht, insbesondere flüssigkeits- und gasdicht, ausgebildet werden kann. Die Dichtheit der Verbindung kann beispielsweise mittels Helium-Überdruckverfahren gemäß DIN EN 1779 nachgewiesen werden. Hierbei kann die Kammer des Behälters mit Helium oder einem heliumhaltigen Gasgemisch beaufschlagt werden, verschlossen und unter Druck gesetzt werden. An der Außenseite des Behälters kann dann mit einem entsprechenden Leckagesuchgerät ein Entweichen nachgewiesen werden. Mittels einer derartigen Prüfung kann demnach die flüssigkeits- und gasdichte Ausbildung der Verbindung von Kanüle und Behältnis geprüft und nachgewiesen werden.

Die Erfindung ermöglicht es, eine Verbindung zwischen Kanüle und Behältnis zu schaffen, die bei einer Druckbeaufschlagung mit bis zu 2 bar, bevorzugt bis zu 3 bar oder darüber hinaus dicht ist. Die Verbindung zwischen Kanüle und Behältnis kann somit als mikrobiologisch dicht verschlossen gewertet werden. Die Ausführung der Verbindung von Kanüle und Behältnis stellt demzufolge eine Sterilitätsbarriere dar, welche eine Langzeitlagerfähigkeit ermöglicht.

Dies führt zu dem großen Vorteil der Erfindung, dass ein mit einem Fluid, beispielsweise mit einem pharmazeutischen oder kosmetischen Präparat vorgefüllter Behälter nach Befüllen und Sterilisieren wenigstens ein Jahr, bevorzugt wenigstens zwei Jahre, besonders bevorzugt wenigstens drei Jahre oder mehr als drei Jahre gelagert werden kann, ohne dass die Stabilität und die Sterilität des Fluids, beispielsweise eines pharmazeutischen oder kosmetischen Präparats, unzulässig beeinträchtig wird.

Die Herstellung eines erfindungsgemäß vorgefüllten Behälters, umfassend ein Behältnis und eine Kanüle, kann dabei auf folgende Weise erfolgen: Die erforderlichen Komponenten werden unter Reinraumbedingungen produziert und montiert, danach sterilisiert und dann steril verpackt. Dieser vorfüllbare Behälter kann dann aseptisch mit dem Fluid, beispielsweise mit einem pharmazeutischen oder kosmetischen Präparat, befüllt werden, um den erfindungsgemäß vorgefüllten Behälter zur Verfügung zu stellen.

Es ist auch möglich, einen vorfüllbaren Behälter im Reinraum mit dem Fluid, insbesondere mit dem pharmazeutischen Präparat, zu befüllen und danach den erfindungsgemäß vorgefüllten Behälter zu sterilisieren.

Zum Applizieren pharmazeutischer oder kosmetischer Präparate sind Behältnisse wie Spritzen, Spritzenkörper, Karpulen oder Injektionsvorrichtungen in unterschiedlichen Größen im medizinischen Bereich bekannt und erhältlich, die für die Erfindung geeignet sind. Derartige Behältnisse sind im Allgemeinen von zylinderförmiger, langgestreckter Form und verfügen über eine zentrale Kammer zur Aufnahme des Fluids. Die Kammer wird dabei axial von einem proximalen und einem distalen Ende begrenzt. Unter dem distalen Ende soll das Ende der Kammer verstanden werden, welches beim Applizieren des Fluids von der Hand der handhabenden Person weg zeigt.

Das proximale Ende der Kammer umfasst dabei typischerweise eine Öffnung, in die ein Stopfen eingesetzt werden kann, um ein eingefülltes Fluid mittels einer Stopfenbewegung, beispielsweise über eine Kolbenstange, hydraulisch oder mittels einer Pumpe angetrieben, in axialer Richtung zu dem entgegengesetzten distalen Ende zu treiben, welches typischerweise eine Spitze mit einem Durchgangskanal zur Kammer umfasst. Ein wichtiges Element ist hierbei das Einsetzen eines Stopfens, der dann entweder mit einer Kolbenstange oder z.B. hydraulisch in Richtung der Kanüle bewegt wird, um dabei beispielsweise ein Medikament abzugeben. Die Spitze kann dabei abgestuft sein oder konisch zulaufend oder auch sich kegelförmig verjüngend ausgebildet sein.

Das Fluid kann dann bei einer entsprechenden Stopfenbewegung durch diesen Durchgangskanal innerhalb der meist abgestuft ausgebildeten Spitze ausgetrieben werden. Die Mittenachse des Behälters stellt die Längsachse der nachfolgend beschriebenen Anordnungen dar.

Sofern nachfolgend von einem Applizieren gesprochen wird, so ist hierunter zum einen ein Injizieren oder Einspritzen eines Fluids, beispielsweise eines pharmazeutischen oder kosmetischen Präparates, mittels des vorgefüllten oder gefüllten Behälters zu verstehen. Applizieren umfasst damit beispielsweise das Injizieren oder Einspritzen eines kosmetischen oder pharmazeutischen Präparates aus dem vorgefüllten Behälter in den menschlichen Körper.

### Austauschseite 18

Es ist aber auch möglich, einen erfindungsgemäß vorfüllbaren Behälter zur Entnahme oder Aufnahme eines Fluids zu verwenden. In diesem Zusammenhang meint der Begriff Applizieren eine Aufnahme eines Fluids in die Kammer.

Ferner umfasst das Applizieren auch eine Verwendung des Behälters zum Dosieren, beispielsweise im Zusammenhang mit einer Infusion. Zusammenfassend tritt beim Applizieren ein Fluid durch den Durchgangskanal in die Kammer ein oder aus der Kammer durch den Durchgangskanal aus.

Zum Verbinden der Kanüle mit dem Behältnis werden erfindungsgemäß eine Kanülenbaugruppe und ein Adapter zu Verfügung gestellt.

Die Kanülenbaugruppe, umfasst
ein zylindersymmetrisches Dichtelement mit einem axialen Durchgang, und
eine Kanüle,
wobei die Kanüle koaxial zum Dichtelement und zumindest abschnittsweise innerhalb des zentrischen Durchgangs des Dichtelements angeordnet ist,
wobei ein Abschnitt der Mantelfläche der Kanüle stoff- und/oder formschlüssig fest mit dem Dichtelement verbunden ist und
wobei zumindest eine Stirnseite des Dichtelements einen den Durchgang umgebenden, kreisringförmigen Bereich umfasst, welcher eine stirnseitige Dichtfläche in einer Ebene senkrecht zur Mittenachse des Dichtelements bildet.

Sofern im Rahmen der Erfindung von einer stoff- und/oder formschlüssigen Verbindung gesprochen wird, so wird mit dem Begriff "stoffschlüssig" ein Zusammenhalt der Verbindungspartner durch atomare oder molekulare Kräfte bezeichnet, und mit dem Begriff "formschlüssig" ein Ineinandergreifen von mindestens zwei Verbindungspartnern.

Das zylindersymmetrische Dichtelement ist mit einem axialen zentrischen Durchgang ausgebildet. Dieser dient der Aufnahme der Kanüle.

Unter einer Kanüle wird nachfolgend eine Hohlnadel oder eine Injektionsnadel mit einem durchgehenden Kanal verstanden, welche vorzugsweise im medizinischen Bereich eingesetzt werden kann.

Der Außendurchmesser der Kanüle wird typischerweise in der Größenangabe Gauge angegeben und ist in den einschlägigen Normen EN ISO 6009 und EN ISO 9626 hinterlegt. Erfindungsgemäß können Kanülen mit einer Gauge-Größe ab dem Wert 10 G bis hin zu einem Wert von 34 G oder auch mit noch geringerem Außendurchmesser eingesetzt werden. Übliche Gauge-Größen, die sich durch Kleben oder durch eine Montage im Spritzgussverfahren montieren lassen, sind 10 G bis 29 G.

### Austauschseite 20

Günstiger für eine patientenfreundliche Applikation eines pharmazeutischen Präparates sind jedoch Kanülen mit noch kleineren Außendurchmessern. So macht die Erfindung die Nutzung von Kanülen von 27 G bis 30 G, bevorzugt 31 G und 32 G und besonders bevorzugt 33 G und 34 G und darüber möglich.

Vorzugsweise kann die Kanüle koaxial zu dem Dichtelement derart angeordnet werden, dass die Kanüle in montierter Position zumindest abschnittsweise innerhalb des zentrischen Durchgangs des Dichtelements angeordnet ist. Dichtelement und Kanüle sind dabei vorzugsweise fest und dicht miteinander verbunden. Die Verbindung zwischen Kanüle und Dichtelement ist vorzugsweise flüssigkeits- und/oder gasdicht ausgebildet.

Die Kanülenbaugruppe kann auf die Spitze des Behältnisses aufgesetzt werden, um die Kanüle radial und/oder axial gegenüber dem Durchgangskanal des Behältnisses zu positionieren.

Zudem kann die Kanülenbaugruppe in montierter Position die erforderliche Dichtung des Durchgangskanal des Behältnisses gewährleisten. Zum Haltern der Kanülenbaugruppe auf der Spitze des Behältnisses und zum Erzeugen der Dichtwirkung wird das Dichtelement daher in montierter Position vorzugsweise mit einer axialen Anpresskraft beaufschlagt und in Richtung der Spitze gepresst. Hierzu wird erfindungsgemäß ein Adapter zur Verfügung gestellt.

Die flüssigkeits- und/oder gasdichte Abdichtung des Durchgangskanals des Behältnisses erfolgt zum einen durch die bereits erwähnte flüssigkeits- und/oder gasdichte Ausbildung der Verbindung zwischen Dichtelement und Kanüle, welche einen Durchtritt des Fluids oder von anderen Substanzen zwischen Dichtelement und Kanüle verhindert, zum anderen durch das stirnseitige Anpressen des Dichtelements auf die den Durchgangskanal umgebende Spitze.

Auf diese Weise kann den Sterilitätsanforderungen für pharmazeutische oder kosmetische Präparate Rechnung getragen werden, wenn diese in die Kammer eingefüllt sind, da das Eindringen von artfremden Substanzen, wie Gasen, Keimen oder auch anderen in die Kammer verhindert werden kann. Zudem wird einem unterwünschten Austritt des Fluids aus der Kammer entgegengewirkt.

Die Kanüle kann hierzu beispielsweise mittels Klemmung und/oder Reibschluss in dem Durchgang des Dichtelements gehaltert sein, d.h. der Innendurchmesser des Durchgangs des Dichtelements kann von Vorteil ein Untermaß gegenüber dem Außendurchmesser der Kanüle in dem zur Befestigung mit dem Dichtelement vorgesehenen Abschnitt aufweisen. Der Innendurchmesser des Durchgangs des Dichtelements ist daher von Vorteil auf den Außendurchmesser der Kanüle abgestimmt.

Alternativ oder in Ergänzung zu einem Klemmen kann die Kanüle mit dem Dichtelement mit einem Verbindungspunkt oder einer Verbindungsfläche stoffschlüssig verbunden sein, beispielsweise mittels Laserschweißen. Aufgrund der festeren Verbindung ist eine Verbindungsfläche von Vorteil, welche durch einen Abschnitt der Mantelfläche der Kanüle mit einem entsprechenden Abschnitt der Innenwandung des Durchgangs gebildet werden kann.

Die Kanüle kann auch ergänzend oder zusätzlich auf der Mantelfläche einen Vorsprung, beispielsweise in Form einer umlaufenden Rippe umfassen, welcher in montierter Position in Eingriff mit einer passgenau gegengleichen Ausnehmung oder Nut, vorzugsweise innerhalb des Durchgangs des Dichtelements, stehen kann und somit noch wirksamer einer axialen Verschiebung der in dem Dichtelement gehalterten Kanüle entgegenwirken kann.

In einer bevorzugten Ausführungsform durchdringt die Kanüle den Durchgang des Dichtelements vollständig. Die Kanüle schließt bündig mit dem Dichtelement ab, zumindest auf derjenigen Seite, welche in montierter Position zu der Kammer hinweist. Im Gebrauch bietet dies den Vorteil, dass bei einem vollständigen Entleeren das Todvolumen in der Kammer möglichst gering gehalten werden kann. Ein geringer Überstand der Kanüle gegenüber dem Dichtelement auf der derjenigen Seite, welche in montierter Position in Richtung Kammer weist, kann dagegen unterstützen, dass die Kammer und die Kanüle einwandfrei miteinander kommunizieren können und somit ein Fluid aus der Kammer in die Kanüle eindringen kann, ohne die Gefahr, dass der Kanüleneingang von dem Dichtelement bedeckt wird. Ein Überstand der Kanüle von etwa 0,1 mm bis hin zu 3 mm, bevorzugt bis zu 2 mm gegenüber der in montierter Position zur Kammer hinweisenden stirnseitigen Dichtfläche erscheint daher günstig.

Der Überstand der Kanüle auf der gegenüberliegenden Seite des Dichtelements bringt den erforderlichen Freiraum der Kanüle gegenüber dem Behältnis, welcher für das Applizieren des Fluids bei Verwendung des erfindungsgemäßen Behälters erforderlich ist. Diese freie Länge der Nadel liegt dabei günstigerweise in einem Bereich von etwa 0,6 cm (2/8 Inch) bis hin zu etwa 5 cm (2 Inch).

Das Dichtelement ist aus einem elastomeren Material gefertigt. Derartige Materialien weisen eine gewisse Nachgiebigkeit bzw. Kompressibilität bei Druckbelastung auf und bieten sich daher zum Abdichten von Öffnungen an. Das erfindungsgemäße Dichtelement kann damit besonders günstig auf den Durchgangskanal der Spitze des Behältnisses aufgesetzt und axial angepresst werden, um die Öffnung abzudichten. Das Dichtelement kann daher, wenn die Kanülenbaugruppe auf dem Behälter montiert ist, den Freiraum fest und dauerhaft dicht verschließen, der sich zwischen der Mantelfläche einer in den Durchgangskanal hineinragenden beziehungsweise an diesen angrenzenden Kanüle und der Innenwandung des Durchgangskanals im Bereich der Spitze einstellt.

Da auf diese Weise das Dichtelement in Kontakt mit einem in die Kammer eingefüllten Fluid, beispielsweise in Kontakt mit einem eingefüllten pharmazeutischen oder kosmetischen Präparat kommen kann, ist es von großem Vorteil, wenn das elastomere Material des Dichtelements für eine Nutzung in Kontakt mit einem pharmazeutischen Präparat zugelassen ist. Auf diese Weise kann einer Kontamination eines eingefüllten Kontakt mit einem pharmazeutischen Präparat zugelassen ist. Auf diese Weise kann einer Kontamination eines eingefüllten Fluids entgegengewirkt werden, so dass eine lange Haltbarkeit und Lagerfähigkeit eines in die Kammer eingefüllten Fluids gegeben ist. Geeignete elastomere Materialien können, beispielsweise ein thermoplastisches Elastomer, insbesondere Butyl-, Chlorbutyl-, Brombutyl- oder Polyisoprenkautschuk umfassen.

Um den Durchgangskanal der typischerweise konisch zulaufenden Spitze des Behältnisses besonders gut abdichten zu können, umfasst zumindest eine Stirnseite des Dichtelements eine den Durchgang des Dichtelements umgebenden, vorzugsweise kreisringförmigen Bereich, welcher eine Dichtfläche in einer Ebene senkrecht zur Mittenachse des Dichtelements bildet. Diese stirnseitige Dichtfläche kann zwar plan vorliegen, muss dieses jedoch nicht und kann beispielsweise konisch sich zur Mitte hin verlängernd ausgeführt sein. Diese Dichtfläche liegt auf derjenigen Seite, welche in montierter Position zu dem Behälter hinweist und mit der Stirnseite des Behälters beziehungsweise mit der Stirnseite der Spitze in Kontakt steht, um diese abzudichten. Die Dichtfläche bildet damit einen Anschlag.

Die geometrische Ausbildung der Oberfläche der stirnseitigen Dichtfläche ist dabei vorzugsweise auf die Form der abzudichtenden Stirnseite der Spitze abgestimmt. Ist beispielsweise die abzudichtende Stirnseite der Spitze als plane Kreisringfläche ausgebildet, so bietet es sich an, die Dichtfläche des Dichtelements ebenfalls plan. konischen Dichtfläche ausgebildet, so kann sich eine gegengleiche Ausbildung der Dichtfläche des Dichtelements als günstig erweisen, beispielsweise eine konkave Form der Oberfläche.

Die stirnseitige Dichtfläche des Dichtelements bildet damit in montierter Position eine umlaufende Kontakt- und Dichtfläche mit der Stirnseite der Spitze und liegt vorzugsweise auf der Stirnfläche der Spitze umlaufend flächig auf und kann zusätzlich in den Zwischenraum zwischen Kanüle und Innenwand einragen und hiermit die Dichtwirkung erhöhen.

In montierter Position kann die Kanüle mit der Kammer des Behälters kommunizieren. Dies bedeutet, dass ein Fluid von der Kammer in die Kanüle oder auch in umgekehrter Richtung bewegt werden kann. Die Kanüle kann in montierter Position in geringem Umfang in den Durchgangskanal der Spitze des Behälters hineinragen, und der sich zwischen der Kanüle und dem Durchgangskanal ergebende Freiraum wird im Stirnbereich der Spitze durch das Dichtelement abgedichtet.

Um diesen Freiraum abdichten zu können, sind von Vorteil der Innen- und der Außendurchmesser des Dichtelements, welche die Dichtfläche außen und innen begrenzen, geometrisch an die Stirnseite des Behälters bzw. an die Spitze angepasst, mit welchem sie in montierter Position in Kontakt stehen.

Um eine vollflächige Abdichtung zu gewährleisten, weist die kreisringförmige Dichtfläche vorzugsweise eine Ausdehnung in radialer Richtung von wenigstens 0,5 mm, bevorzugt von wenigstens 0,7 mm und besonders bevorzugt von wenigstens 1 mm auf. In einer bevorzugten Ausführungsform weist die kreisringförmige Dichtfläche eine Ausdehnung in radialer Richtung auf, welche wenigstens der radialen Ausdehnung der stirnseitigen Fläche der Spitze entspricht. Eine leichte radiale Überdeckung dieser stirnseitigen Fläche durch die kreisringförmige Dichtfläche nach innen und/oder außen kann dabei von Vorteil sein, um eine möglichst breite Dichtfläche als Kontaktfläche zwischen Dichtelement und Spitze in montierter Position zu schaffen.

Vorteilhaft ist es, wenn der Innendurchmesser des Durchgangs des Dichtelements kleiner oder zumindest nicht größer ist als der Innendurchmesser des Durchgangskanals des Behältnisses, wobei der Innendurchmesser des Durchgangs des Dichtelements sich aber letztendlich nach der Gauge-Größe der Kanüle richtet.

Weiterhin ist es günstig, wenn der Außendurchmesser des Dichtelements korrespondierend zu dem Außendurchmesser der Spitze ausgebildet ist, um einen Überstand gegenüber der Spitze zu vermeiden.

Gängige Größen von erfindungsgemäß geeigneten Behältnissen und Spitzen sind in der Norm ISO 80369-7 angegeben.

Um die Dichtwirkung weiter zu erhöhen, kann alternativ oder zusätzlich die stirnseitige Dichtfläche mit einem zentrischen, vorzugsweise kegelstumpfförmigen Vorsprung ausgebildet sein. Dieser kann den zentrischen Durchgang des Dichtelements umgeben und in montierter Position zumindest in seinem stirnseitigen Bereich in den Durchgangskanal der Spitze eingreifen beziehungsweise hineinragen. Auf diese Weise kann besonders günstig eine weitere Abdichtung zwischen Spitze und Dichtelement geschaffen werden, wobei sich der Vorteil ergibt, dass die Abdichtung zwischen Dichtelement und Durchgangskanal bereits in dem Durchgangskanal erfolgt, so dass kein Fluid in den Spalt zwischen den Stirnseiten von Spitze und Dichtelement gelangen kann. Der Vorsprung ermöglicht auch eine bessere Führung und/oder Positionierung der Kanüle in dem Durchgangskanal und/oder relativ zu dem Behältnis.

Der vorzugsweise kegelstumpfförmige Vorsprung kann von Vorteil ebenfalls geometrisch an den Durchgangskanal des zu verschließenden Behälters angepasst sein. Es hat sich als vorteilhaft herausgestellt, wenn dessen Länge in axialer Richtung so bemessen ist, dass der Vorsprung in montierter Position wenigstens 0,1 mm, bevorzugt wenigstens 1 mm und besonders bevorzugt wenigstens 2 mm, und nicht mehr als 5 mm, bevorzugt nicht mehr als 4 mm und besonders bevorzugt nicht mehr als 3 mm in den Durchgangskanal hineinragt.

Um eine gute Dichtwirkung zu erhalten, beträgt der Winkel zwischen der Achse des kegelstumpfförmigen Vorsprungs und der Mantellinie wenigstens 1°, bevorzugt wenigstens 8° und besonders bevorzugt wenigstens 10° bis 30°.

Vorteilhaft ist hierbei auch die Selbstjustierung bei der Montage von Kanülenbaugruppe und Behältnis.

Erfindungsgemäß ist vorgesehen, die Kanülenbaugruppe, umfassend Kanüle und Dichtelement, fest und dauerhaft dicht mit der Spitze des Behältnisses, insbesondere einer Spritze, einem Spritzenkörper, einer Karpule oder einer Injektionsvorrichtung, zu verbinden, um den erfindungsgemäßen vorfüllbaren und/oder vorgefüllten Behälter zum Lagern und/oder Applizieren eines Fluids, beispielsweise eines pharmazeutischen oder kosmetischen Präparates, zur Verfügung zu stellen.

Hierzu wird in einem weiteren Aspekt der Erfindung ein Adapter zum festen und dauerhaft dichten Verbinden einer Kanüle, insbesondere eine Kanülenbaugruppe, mit der Spitze eines Behältnisses, insbesondere einer Spritze, einem Spritzenkörper, einer Karpule oder einer Injektionsvorrichtung vorgeschlagen. Dieser Adapter ermöglicht es, eine Kanüle fest und dauerhaft dicht mit dem Behältnis derart zu verbinden, dass die oben genannten Anforderungen insbesondere im Hinblick auf die Lagerfähigkeit und/oder die Sterilitätsanforderungen eingehalten werden können.

Der erfindungsgemäße Adapter ermöglicht es auf besonders einfache Weise, die axiale und/oder radiale Kompression zur Verfügung zu stellen, die erforderlich ist, um die Kanülenbaugruppe fest und dauerhaft dicht mit Behältnis zu verbinden.

Um derartige Behältnisse, insbesondere eine Spritze, einen Spritzenkörper, eine Karpule oder einer Injektionsvorrichtung füllen, verschließen und wieder öffnen zu können, sind für den medizinischen Bereich unter anderem Verbindungssysteme entwickelt worden, die auf einer sogenannten Luer-Lock-Verbindung beruhen. So sind Behältnisse zum Lagern und Applizieren pharmazeutischer Präparate erhältlich, welche mit einem ersten Teil einer Luer-Lock-Verbindung ausgestattet sind, und welche mit einem Verschluss, umfassend einen zweiten Teil einer Luer-Lock-Verbindung, verschlossen und zum Applizieren eines Präparates später wieder geöffnet werden können. Diese auf dem Luer-Lock-Prinzip basierende Art der Verbindung macht sich auch die Erfindung zunutze.

Eine Luer-Lock-Verbindung umfasst dabei ein Verbindungselement mit einem Innengewinde als erstes Teil der Luer-Lock-Verbindung, in das ein zweites Verbindungselement mit einem Außengewinde als zweites Teil eingreifen und mit einer Drehbewegung um eine Drehachse befestigt werden kann. Dass das Innengewinde aufweisende Verbindungselement wird auch als männlicher Teil einer Luer-Lock-Verbindung und das das Außengewinde aufweisende Verbindungselement auch als weiblicher Teil einer Luer-Lock-Verbindung bezeichnet.

Die Erfinder haben herausgefunden, dass ein derartiges, zum Verschließen und Öffnen eines Behälters dienendes Verbindungsprinzip sich besonders einfach auch zum Verbinden der Kanüle mit dem Behältnis eignet.

Gegenstand der Erfindung ist demnach in einem weiteren Aspekt ein Adapter zum Verbinden einer Kanüle, insbesondere einer Kanülenbaugruppe, mit einem Behältnis, insbesondere mit der Spitze eines Behältnisses wie einer Spritze, eines Spritzenkörpers, einer Karpule oder einer Injektionsvorrichtung, wobei der Adapter als langgestreckten Hohlkörper ausgebildet ist mit einer axialen Durchgangsöffnung, einem proximalen Ende und einem distalen Ende,
wobei der Innendurchmesser der Durchgangsöffnung an dem proximalen Ende erweitert ist und eine Aufnahme zur Halterung des Dichtelements bildet,
und wobei das proximale Ende ferner ein Außengewinde als Teil einer Luer-Lock-Verbindung umfasst, welches mit dem Innengewinde des Behältnisses verbunden ist oder verbunden werden kann.

Die Begriffe "proximal" und "distal" bezeichnen dabei diejenigen Stirnseiten des Adapters, welche in montierter Position mit denjenigen des Behältnisses, also beispielsweise der Spritze, korrespondieren. In montierter Position weist beispielsweise das proximale Ende des Adapters zu dem Anwender hin.

Die axiale Durchgangsöffnung ist demnach mit zumindest zwei unterschiedlichen Innendurchmessern ausgebildet, wobei derjenige Abschnitt mit dem erweiterten Innendurchmesser sich zu derjenigen Seite des Adapters hin öffnet, welche in montierter Position zu dem Behältnis hinweist.

Dieser erweiterten Innendurchmesser ist von Vorteil geometrisch auf den Außendurchmesser des aufzunehmenden Dichtelements der Kanülenbaugruppe abgestimmt und ermöglicht es somit, das Dichtelement der Kanülenbaugruppe von der Stirnseite aus zumindest teilweise in den Adapter axial einschieben zu können bis zu einem Anschlag, der durch den Übergang der im Durchmesser erweiterten Durchgangsöffnung zu der Durchgangsöffnung geringeren Durchmessers gebildet wird und der hierdurch ein weiteres Einschieben des Dichtelements verhindert.

Der kleinere Innendurchmesser ist derart gewählt, dass die Kanüle in montierter Position diesen Bereich der Durchgangsöffnung des Adapters durchdringen kann, wohingegen das Dichtelement in der vorgesehenen Position gehaltert wird.

Alternativ oder zusätzlich kann aber auch eine weitere formschlüssige, d.h. durch Ineinandergreifen von mindestens zwei Verbindungspartnern entstehende Verbindung zwischen der Innenwandung des Durchgangs und dem Dichtelement geschaffen werden, beispielsweise durch eine umlaufende Nut, welche derart dimensioniert sein kann, dass ein äußerer Abschnitt des Dichtelements in montierter Position in dieser Nut einrasten kann. Auf diese Weise kann das Dichtelement in dem Adapter derart gehaltert werden, dass ein axialer Versatz des Dichtelements relativ zu dem Adapter in beide Richtungen verhindert wird.

In einer weiteren Ausführungsform ist vorgesehen, die Verbindung zwischen Kanülenbaugruppe und Adapter auch stoffschlüssig, d.h. durch atomare oder molekulare Kräfte zusammengehaltene Verbindungspartner, auszubilden.

Bevor der Adapter auf das Behältnis aufgesetzt und verschraubt wird, wird vorzugsweise die Kanülenbaugruppe in den Adapter eingesetzt.

Das proximale Ende des Adapters ist von Vorteil ferner mit einem Außengewinde zum festen Verbinden mit dem Behältnis vorgesehen. Besonders günstig ist dieses Außengewinde als (weiblicher) Teil einer Luer-Lock-Verbindung ausgebildet und ermöglicht es auf diese Weise, mit einem (männlichen) Teil einer Luer-Lock-Verbindung des Behältnisses verbunden zu werden, welches ein entsprechendes Innengewinde aufweist.

Auf diese Weise kann der Adapter dazu verwendet werden, eine Kanüle mit dem Behältnis zu verbinden und dabei die distale Öffnung des Behältnisses abzudichten. Ein großer Vorteil liegt darin, dass der erfindungsgemäße Adapter mit einer Vielzahl von am Markt erhältlichen Behältnissen, insbesondere Spritzen, Spritzenkörpern, Karpulen oder Injektionsvorrichtungen, welche mit einem (männlichen) Luer-Lock-Anschluss mit Innengewinde ausgestattet sind, verwendet werden kann.

Selbstverständlich ist es auch möglich, den Adapter mit anderen Gewindeformen auszustatten und auf diese Weise mit Behältnissen zu verwenden, welche über Gewinde verfügen, die nicht dem Luer-Lock-Prinzip entsprechen.

Zum Verbinden und Verschließen kann der Adapter, vorzugsweise mit bereits eingesetzter Kanülenbaugruppe, auf den Luer-Lock-Anschluss des Behältnisses aufgesetzt und mit einer Drehbewegung aufgeschraubt werden. Der Anschlag in der Durchgangsöffnung ist vorzugsweise derart ausgelegt, dass bei einem aufgeschraubten Verschlusssystem in der vorgesehenen Endstellung das Dichtelement mit der vorbestimmten axialen und/oder radialen Anpresskraft gegen die Spitze des Behältnisses gepresst wird, wodurch die gewünschte Dichtwirkung eintritt und der Durchgangskanal des Behältnisses gegenüber der Kanüle abgedichtet wird.

Zum Abdichten der Kanüle selbst, welche in einer bevorzugten Ausführungsform in montierter Position an dem distalen Ende aus dem Adapter hervorragt, ist, um die geforderte sterile Langzeitlagerfähigkeit zu erreichen, ein weiterer Schutz vorgesehen, welcher dieses Ende der Kanüle sichert und abdichtet.

Erfindungsgemäß ist daher ferner eine aufsteckbare Verschlusskappe vorgesehen, welche beispielsweise einen harten äußeren Bereich und einen abdichtenden, elastomeren inneren Bereich umfassen kann. Die Verschlusskappe kann auf das distale Ende des Adapters aufgesteckt werden und dabei mit dem abdichtenden, elastomeren inneren Bereich den überstehenden Teil der Kanüle (40) zumindest abschnittsweise umfassen und hierdurch abdichten. Der Adapter ist daher in einer bevorzugten Ausführungsform an seinem distalen Ende mit einem Ansatz zum Aufstecken und Haltern einer Verschlusskappe ausgebildet.

Der vorfüllbare oder vorgefüllte Behälter umfasst demnach in einer besonders bevorzugten Ausführungsform eine Verschlusskappe, die dazu ausgebildet ist, in aufgesetzter Position den Kanal der Kanüle gegenüber der Umgebung abzudichten, um die stabile und sterile Langzeitlagerfähigkeit zu gewährleisten. Hierzu kann beispielsweise beim Aufstecken der Verschlusskappe die Kanüle in das abdichtende, elastomere Material im inneren Bereich der Verschlusskappe hineingeschoben werden, so dass das elastomere Material einen leichten Druck in axialer und/oder radialer Richtung auf die Öffnung der Kanüle ausübt, wenn die Verschlusskappe aufgesetzt ist, und diese damit verschließt.

Der Adapter und die Kanülenbaugruppe können auch gemeinsam als Baugruppe gefertigt und gelagert werden und dann mit dem Behältnis verbunden werden. Auf diese Weise stellen sie ein Verschlusssystem für das Behältnis dar, welches flexibel konfiguriert werden kann, beispielsweise mit unterschiedlichen Kanülen. In einer vorteilhaften Ausbildung kann dieses Verschlusssystem die vorstehend genannte Verschlusskappe bereits mit umfassen.

In einer vorteilhaften Weiterbildung der Erfindung ist vorgesehen, den Adapter und/oder die Verschlusskappe in einer bestimmten Farbgebung zu gestalten, welche in Abhängigkeit von zumindest einer Eigenschaft der Kanüle ausgewählt wird, vorzugsweise in Abhängigkeit von dem Außendurchmesser, dem Innendurchmesser, der Länge und/oder der Art des Schrägschliffs der Kanüle.

So ist zum Beispiel ein Farbsystem in der Norm EN ISO 6009 festgelegt, welches eine Farbe in Abhängigkeit von der Gauge-Größe der Kanüle bestimmt. Besonders günstig kann auf diese Weise die Farbgebung des Adapters und/oder die Farbe der Verschlusskappe genutzt werden, um einem Benutzer direkt eine Information über den Außendurchmesser der Kanüle zu geben. Hierdurch können die Sicherheit und die Zugriffszeit verbessert werden.

In einer bevorzugten Ausführungsform richtet sich daher die Farbe des Adapters und/oder der Verschlusskappe nach der Farbcodierung gemäß der Norm EN ISO 6009, um dem Benutzer eine Information über den Außendurchmesser der Kanüle und damit die Gauge-Größe zu geben. Auf diese Weise kann ein Benutzer eines vorfüllbaren oder vorgefüllten erfindungsgemäßen Behälters rasch Informationen über die Kanüleneigenschaften erhalten und den für den konkreten Anwendungsfall erforderlichen Behälter sehr leicht und zuverlässig auswählen.

Auch eine kombinierte Farbgebung ist möglich, beispielsweise eine erste Farbgebung des Adapters, um dem Benutzer eine Information über den Außendurchmesser bzw. die Gauge-Größe der eingebauten Kanüle zu geben, und eine zweite Farbgebung der Verschlusskappe, welche beispielsweise Informationen über die Art des Schrägschliffes oder die Länge der Kanüle gibt.

Das erfindungsgemäße Verschlusssystem kann, vorzugsweise mit bereits eingesetzter Kanülenbaugruppe, auf oder an einem vorfüllbaren oder vorgefüllten Behälter zum Lagern und Applizieren eines Fluids, insbesondere eines pharmazeutischen oder kosmetischen Präparates, verwendet werden, um den Durchgangskanal des Behältnisses abzudichten und eine Kanüle zu befestigen.

Auf diese Weise kann sehr einfach ein vorfüllbarer oder vorgefüllter Behälter, insbesondere eine vorfüllbare oder vorgefüllte Spritze, ein vorfüllbarer oder vorgefüllter Spritzenkörper, eine vorfüllbare oder vorgefüllte Karpule oder eine vorfüllbare oder vorgefüllte Injektionsvorrichtung "ready-to-use" zum Lagern und/oder Applizieren eines Fluids, insbesondere eines pharmazeutischen oder kosmetischen Präparates, zur Verfügung gestellt werden.

Als erfindungsgemäß geeignetes Behältnis kommen demnach eine Vielzahl von am Markt erhältliche Spritzen, Spritzenkörper, Karpulen oder Injektionsvorrichtungen in Betracht, welche vorzugsweise mit einem (männlichen) Luer-Lock-Anschluss mit Innengewinde ausgestattet sind.

Der große Vorteil der Erfindung liegt darin, dass diese Behältnisse kostengünstig in hohen Stückzahlen gefertigt werden können, ohne dass bereits eine passende Kanüle während der Herstellung des Behälters bereitgestellt und mit diesem verbunden werden muss.

Da derartige Behältnisse häufig aus Kunststoff gefertigt und im Spritzgussverfahren hergestellt werden, vereinfacht dies die Produktion ungemein. Zudem ist auch das Handling während der Produktion deutlich einfacher, da keine Behältnisse mit Kanüle prozessiert werden müssen.

Zudem ist es auch sehr viel einfacher, eine große Variantenvielfalt in Bezug auf Behältergröße und Kanüle zur Verfügung zu stellen, ohne dass eine aufwendige Lagerhaltung erforderlich ist. Dies ist dem Umstand geschuldet, dass erfindungsgemäß Behältnis und Kanüle unabhängig voneinander gefertigt, gelagert, transportiert und gemäß der vorgesehenen Verwendung miteinander verbunden werden können.

So können Behältnisse mit einem Volumen von beispielsweise 0,25 ml, 0,5 ml, 0,6 ml, 1,0 ml, 1,2 ml, 1,5 ml, 2,0 ml, 2,25 ml, 3,0 ml, 5,0 ml, 10,0 ml, 20 ml oder 50 ml, sehr einfach mit Kanülen verschiedener Gauge-Größen, z.B. 27, 28, 29, 30 G und insbesondere mit Kanülen mit sehr geringen Außendurchmessern mit Gauge-Größen 31, 32, 33, oder 34 G kombiniert werden.

Neben der Lagerhaltung können auch die Kosten für Werkzeuge reduziert werden, da die Behälter unabhängig von den Dimensionen der Kanülen gefertigt werden können.

Erfindungsgemäß geeignete Behälter können einstückig aus einem transparentem Material gefertigt sein, vorzugsweise aus Glas oder Kunststoff, wobei Kunststoff einen thermoplastischen oder duroplastischen Kunststoff umfassen kann.

Selbstverständlich ist es auch möglich, das Behältnis aus mehr als einem Material zu fertigen, wie es beispielsweise in der Druckschrift DE 10 2014 211 018 A1 beschrieben ist, und deren Inhalt diesbezüglich auch zum Gegenstand der vorliegenden Anmeldung gemacht wird. Die hier beschriebene Ausführungsform eines Behältnisses für pharmazeutische Präparate ermöglicht es, die sehr guten inerten Eigenschaften von Glas in Kontakt mit anderen Substanzen zu nutzen, und gleichzeitig eine Verbindung mit einem Luer-Lock-Anschluss zu ermöglichen, wobei ein Anschlusskörper aus einem Kunststoffmaterial zur Verfügung gestellt wird.

Die thermoplastischen Kunststoffe können die im medizinischen Bereich gebräuchlichen und zugelassenen Kunststoffe umfassen, demzufolge kann der thermoplastische Kunststoff ein Cyclo-Olefin-Copolymer (COC), Cyclo-Olefin-Polymer (COP), Acrylnitril-Butadien-Styrol (ABS), Polyamid (PA), Polylactat (PLA), Polymethylmethacrylat (PMMA), Polycarbonat (PC) oder Polyethylenterephtalat (PET) sein oder diesen umfassen.

Geeignete, im medizinischen Bereich gebräuchliche und zugelassene duroplastische Kunststoffe können ein Celluloseacetat (CA) oder ein transparentes duroplastisches Harz umfassen oder aus diesem Bestehen.

Ein geeignetes Glas kann ein Borosilikatglas sein, welches die folgende Zusammensetzung (in Gew.-%) aufweisen kann:

| | |
|---|---|
| SiO₂ | 65% bis 82% |
| B₂O3 | 5% bis 13% |
| ∑ Na₂O + K₂O | 4% bis 10% |
| Al₂O₃ | 2% bis 9% |

Σ CaO + MgO + BaO + SrO 0% bis 5%

Bevorzugt kann ein solches Glas eine Zusammensetzung (in Gew.-%)aufweisen:

| | |
|---|---|
| SiO₂ | 73% bis 79% |
| B₂O3 | 9% bis 12% |
| Na₂O | 5% - 8% |
| K₂O | 0% bis 0,5% |
| Li₂O | 0% - 0,3 % |
| Al₂O₃ | 3,5% bis 7% |
| CaO | 0% - 3% |
| BaO | 0% - 1% |
| F | 0 - 0,3% |

Σ CaO + MgO + BaO + SrO 0% bis 3%

In einer weiteren Ausführungsform kann ein solches Glas eine Zusammensetzung (in Gew.-%) aufweisen:

| | |
|---|---|
| SiO₂ | 71% bis 77% |
| B₂O3 | 9% bis 12% |
| Na₂O | 5,5% - 8,5% |
| K₂O | 0,1% bis 1,0% |
| Li₂O | 0% - 0,3 % |
| Al₂O₃ | 5,5% bis 8% |
| CaO | 0% - 1,5% |
| BaO | 0% - 1% |
| F | 0 - 0,3% |

Σ CaO + MgO + BaO + SrO 0% bis 2%

Der erfindungsgemäße vorfüllbare oder vorgefüllte Behälter zeichnet sich weiterhin dadurch aus, dass in montierter Position die Anpresskraft des Dichtelements, insbesondere auf die Stirnseite der Spitze wenigstens 0,1 bis 30 N, bevorzugt 5 bis 20 N beträgt. Weiterhin zeichnet sich der erfindungsgemäße vorfüllbare Behälter dadurch aus, dass in montierter Position der Anpressdruck des Dichtelements in der Verschlusskappe auf das Ende der Kanüle wenigstens 20 N, bevorzugt wenigstens 30 N beträgt.

Aufgrund des Zusammenwirkens beider Abdichtungen wird hinsichtlich der Stabilität und im Falle einer Sterilisierung des Behälters auch der Sterilität eine Langzeitlagerung ermöglicht.

Um die Dichtwirkung noch weiter zu verbessern, kann alternativ oder zusätzlich die stirnseitige Dichtfläche mit einem zentrischen, vorzugsweise kegelstumpfförmigen Vorsprung ausgebildet sein und steht in einer bevorzugten Ausführungsform der vorzugsweise kegelstumpfförmige Vorsprung des Dichtelements in montierter Position in Wirkzusammenhang mit dem Durchgangskanal der Spitze des Behältnisses. In anderen Worten, der Vorsprung des Dichtelements ragt zumindest abschnittsweise in den Durchgangskanal des abzudichtenden Behältnisses hinein. Gute Abdichtwirkungen lassen sich erzielen, wenn der Vorsprung wenigstens 0,1 mm, bevorzugt wenigstens 1 mm und besonders bevorzugt wenigstens 2 mm, und nicht mehr als 5 mm, bevorzugt nicht mehr als 4 mm und besonders bevorzugt nicht mehr als 3 mm in den Durchgangskanal hineinragt.

Der in den Durchgangskanal eingreifende Vorsprung führt zu einem weiteren Vorteil, da er such das Dichtelement in dessen Lage sichert. Auch eine halbreisförmige Ausstülpung ist möglich und kann im Wesentlichen zur Zentrierung im Durchgangskanal verwendet werden.

Die Erfindung betrifft demnach auch einen vorgefüllten Behälter, umfassend eine Spritze, einen Spritzenkörper, eine Karpule oder eine Injektionsvorrichtung, mit einer sehr dünnen Kanüle zum Injizieren eines Fluids, beispielsweise eines pharmazeutischen oder kosmetischen Präparates. Das kosmetische Präparat kann beispielsweise eine kosmetische Zubereitung umfassen.

Die Erfindung ermöglicht es damit auf besonders einfache Weise, ein Behältnis mit einer Kanüle einer Gauge-Größe von größer 30 G, bevorzugt von 31 G, 32 G, 33 G oder 34 G zu verbinden.

Der vorgefüllte Behälter kann mit einem Fluid oder mit einer Flüssigkeit gefüllt sein, beispielsweise mit einem pharmazeutischen Präparat oder mit einer vorzugsweise flüssigen, kosmetischen Zubereitung. Erfindungsgemäß kann somit auch ein vorgefüllter Behälter mit einem Fluid oder einer Flüssigkeit, bevorzugt mit einer kosmetischen Zubereitung, zur Verfügung gestellt werden, welcher dadurch gekennzeichnet ist, dass die Kanüle 40 eine Gauge-Größe von größer 30 G, bevorzugt von 31 G, 32 G, 33 G oder 34 G aufweist.

Erfindungsgemäß ist vorgesehen, dass ein vorgefüllter erfindungsgemäßer Behälter nach Befüllen wenigstens ein Jahr, bevorzugt wenigstens zwei Jahre, besonders bevorzugt wenigstens drei Jahre oder sogar mehr als drei Jahre gelagert werden kann, wobei die geltenden Sterilisationsanforderungen erfüllt werden.

Hierzu ist es vorteilhaft, wenn die vorstehend beschriebenen Baugruppen röntgen- oder gammastrahlenstabil ausgeführt und somit einer Röntgen- oder Gammastrahlensterilisierung zugänglich sind, welche eine Verkeimung des vorfüllbaren oder vorgefüllten Behälters sicher ausschließt.

In weiterer vorteilhafter Ausführung sind die vorstehend beschriebenen Baugruppen bis 121 °C temperaturstabil und können folglich auch autoklaviert werden.

Je nach Präparat kann dann auch ein befüllter, insbesondere vorbefüllter Behälter zusammen mit dem Präparat sterilisiert werden, welches erheblich zu einer verbesserten Lagerfähigkeit beitragen kann, da hierbei Keime innerhalb des Behälters in der Regel mit hoher Sicherheit ausgeschlossen werden können.

Um beispielsweise Manipulationen an einem mit dem erfindungsgemäßen Verschlusssystem vorgefüllten Behälter zu verhindern, ist in einer Weiterbildung der Erfindung vorgesehen, die formschlüssige, d.h. durch Ineinandergreifen von mindestens zwei Verbindungspartnern entstehende Luer-Lock-Verbindung mit zusätzlichen Applikationen auszustatten, welche einem unerwünschten Öffnen, oder einem Öffnen und Wiederverschließen und/oder sonstigen Manipulationen entgegenwirken.

So kann in einer Ausführungsform eine Ummantelung in Form einer Versiegelung oder Banderole vorgesehen sein, welche zumindest Abschnitte der Mantelfläche des Adapters und der Mantelfläche des Behälters umgibt und miteinander verbindet. Die Ummantelung kann so ausgebildet sein, dass eine Drehbewegung des Adapters zum Öffnen des Behälters zu einer Beschädigung der Ummantelung führt, welche für einen Benutzer sichtbar wird. Auf diese Weise lässt sich verhindern, dass der Behälter durch Drehen des Verschlusssystems geöffnet und ggf. auch wieder verschlossen wird. Ein einmal geöffneter Behälter kann auf diese Weise einfach erkannt werden.

In einer anderen oder ergänzenden Ausführungsform kann vorgesehen sein, die durch Drehbewegung lösbare Luer-Lock-Verbindung zu einer nicht lösbaren Luer-Lock-Verbindung weiter zu entwickeln. Dies kann beispielsweise dergestalt erfolgen, dass zusätzlich zu der Luer-Lock-Verbindung eine stoffschlüssige, also eine durch atomare oder molekulare Kräfte zusammengehaltene Verbindungspartner entstehende Verbindung zwischen Verschlusssystem und Behälter geschaffen wird, welche vorzugsweise mit der Luer-Lock-Verbindung zusammenwirkt oder diese ergänzt. Hierzu kann ein stoffschlüssiger Verbindungspunkt oder eine stoffschlüssige Verbindungsfläche zwischen dem Außen- und dem Innengewinde beispielsweise durch Aufbringen eines Klebers, durch Laserschweißen oder durch UV-Schweißen geschaffen werden, wobei diese zusätzliche Verbindung dann aushärtet, wenn der Behälter verschlossen ist.

In überraschend einfacher Weise kann die Luer-Lock-Verbindung somit zu einer unlösbaren Verbindung gemacht werden. Somit kann sichergestellt werden, dass ein mit einem Fluid vorgefüllter Behälter und damit auch das eingefüllte Fluid nicht manipuliert werden können.

Weitere Einzelheiten der Erfindung ergeben sich aus der Beschreibung der dargestellten Ausführungsbeispiele und den angefügten Ansprüchen.

### Die Zeichnungen zeigen:

- Fig. 1: eine Schnittansicht eines Behältnisses, eine Kanülenbaugruppe, umfassend ein Dichtelement und eine Kanüle, einen Adapter und eine Verschlusskappe, als wesentliche Komponenten eines erfindungsgemäßen vorfüllbaren oder vorgefüllten Behälters in einem bevorzugten Ausführungsbeispiel darstellend, wobei die Schnittebene in etwa mittig durch die vorstehend erwähnten Bestandteile verläuft und deren Mittellinie umfasst,
- Fig. 2: eine Schnittansicht eines Behältnisses mit einer aufgeschobenen Kanülenbaugruppe, umfassend ein Dichtelement und eine Kanüle, wobei die Schnittebene in etwa mittig durch diese Bestandteile verläuft und deren Mittellinie umfasst,
- Fig. 3: eine Schnittansicht der Kanülenbaugruppe aus Fig. 2, wobei die Schnittebene in etwa mittig durch die Kanülenbaugruppe verläuft und deren Mittellinie umfasst,
- Fig. 4: eine Schnittansicht eines Adapters eines bevorzugten Ausführungsbeispiels, wobei die Schnittebene in etwa mittig durch den Adapter verläuft und dessen Mittellinie umfasst,
- Fig. 5: eine Schnittansicht eines Verschlusssystems, umfassend eine Kanülenbaugruppe nach Fig. 3 und einen Adapter nach Fig. 4 sowie eine Verschlusskappe, wobei die Schnittebene in etwa mittig durch diese Bestandteile verläuft und deren Mittellinie umfasst,
- Fig. 6: ein Behältnis am Beispiel einer Spritze in einer Schnittansicht, wobei die Schnittebene in etwa mittig durch das Behältnis verläuft und dessen Mittellinie umfasst, und
- Fig. 7: einen vorfüllbaren Behälter eines bevorzugten Ausführungsbeispiels, umfassend ein Behältnis, wie dieses in Fig. 6 dargestellt ist, sowie ein Verschlusssystem, wie dies in Fig. 5 dargestellt ist, in einer Schnittansicht, bei welcher die Schnittebene in etwa mittig durch das Behältnis verläuft und dessen Mittellinie umfasst.

### Detaillierte Beschreibung bevorzugter Ausführungsformen

Bei der nachfolgenden detaillierten Beschreibung bevorzugter Ausführungsformen bezeichnen um der Klarheit willen gleiche Bezugszeichen im Wesentlichen gleiche Teile in oder an diesen Ausführungsformen. Zur besseren Verdeutlichung der Erfindung sind die in den Figuren dargestellten bevorzugten Ausführungsformen jedoch nicht immer maßstabsgerecht gezeichnet.

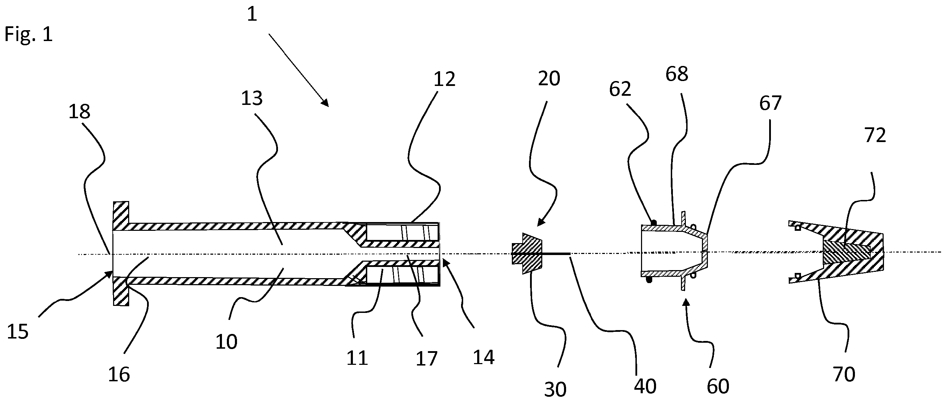

In Fig. 1 sind in einer Schnittansicht in einem Ausführungsbeispiel abgebildet ein Behältnis 10, eine Kanülenbaugruppe 20, umfassend ein Dichtelement 30 und eine Kanüle 40, ein Adapter 60 und eine Verschlusskappe 70, als wesentliche Komponenten des erfindungsgemäßen vorfüllbaren oder vorgefüllten Behälters 1 zum Lagern und/oder Applizieren eines Fluids, insbesondere eines pharmazeutischen oder kosmetischen Präparates.

Das Behältnis 10 ist in dem abgebildeten Beispiel ohne Beschränkung auf das abgebildete Ausführungsbeispiel eine Spritze mit Luer-Lock-Anschluss mit Innengewinde und einem Kammervolumen von 10 ml.

Insgesamt wird ein vorfüllbarer oder vorgefüllter Behälter 1 zum Lagern und/oder Applizieren eines Fluids, insbesondere eines pharmazeutischen oder kosmetischen Präparates, offenbart umfassend ein Behältnis 10, insbesondere eine Spritze, mit einem Spritzenkörper, eine Karpule oder eine Injektionsvorrichtung, mit einer Kammer 13 zur Aufnahme des Fluids, ferner umfassend eine Kanülenbaugruppe 20, wobei die Kanülenbaugruppe eine Kanüle 40 und ein Dichtelement 30 aufweist und die Kanülenbaugruppe 20 an dem Behältnis 10 anbringbar ist, sowie umfassend eine Verschlusskappe 70, wobei eine außenseitige Kanalöffnung 43 der Kanüle 40 in einen abdichtenden, elastomeren Innenbereich 72 der Verschlusskappe 70 hineinragt und dieser die Kanalöffnung 43 abdichtet, wobei durch eine radiale und/oder axiale Kompression des Dichtelements 30 sowie durch die Abdichtung der Kanalöffnung 43 mittels der Verschlusskappe 70 die Kammer 13 fluiddicht verschlossen ist.

Es wird mit der Erfindung folglich auch eine formatflexible, kanülenflexible, vormontierte, vorgefüllte Spritze mit Luer-Lock, bzw. eine Stack-Needle mit Adapter auf dem Luer-Lock vormontiert, mit langzeitdichter Abdichtung offenbart, welche in einer Art Modulbauweise alle Varianten von Kanülen und insbesondere gerade die sehr dünnen (von beispielsweise 31 bis 34 G), auf einfache Weise im System integrierbar macht.

Der erfindungsgemäße Adapter 60 und die erfindungsgemäße Kanülenbaugruppe 20 sind erfindungsgemäß mit unterschiedlichen Behältnissen 10 verwendbar, umfassend Spritzen, Spritzenkörper, Karpulen oder Injektionsvorrichtungen mit verschiedenen Kammervolumen, vorzugsweise in einem Bereich von 0,6 ml bis 50 ml, sowohl aus Glas als auch aus Kunststoff. Das Behältnis 10 in dem Ausführungsbeispiel verfügt über einen Luer-Lock-Anschluss mit einem Innengewinde 11.

Die Kanülenbaugruppe 20 und der Adapter 60 dienen dazu, die Kanüle 40 mit dem Behältnis 10 zu verbinden, um den erfindungsgemäßen vorfüllbaren oder vorgefüllten Behälter 1 zum Lagern und/oder Applizieren eines Fluids, beispielsweise eines pharmazeutischen oder kosmetischen Präparates, zur Verfügung zu stellen. Der Behälter 1 kann mit dem Fluid gefüllt und der vorgefüllte Behälter gelagert werden, bevor mittels der Kanüle 40 das Fluid appliziert werden kann. Das Behältnis 10 ist in dem Ausführungsbeispiel von zylinderförmiger, langgestreckter Form und verfügt über eine zentrale Kammer 13 zur Aufnahme des Fluids. Die Kammer 11 wird dabei axial von einem proximalen Ende 15 und einem distalen Ende 14 begrenzt. Das distale Ende 14 weist beim Applizieren des Fluids von der Hand der handhabenden Person weg.

Die Bezeichnungen distal und proximal werden analog auch für die weiteren Komponenten des erfindungsgemäßen Behälters 1 verwendet und beziehen sich auf die entsprechenden Seiten oder Richtungen dieser Komponenten in montierter Position.

Das proximale Ende 15 der Kammer 11 umfasst dabei eine Öffnung 16, in die ein Stopfen eingesetzt werden kann, um ein eingefülltes Fluid mittels einer Stopfenbewegung, beispielsweise über eine Kolbenstange oder hydraulisch angetrieben, in axialer Richtung zu dem entgegengesetzt angeordneten distalen Ende 14 aus der Kammer 11 zu treiben. Durch eine umgekehrte Bewegung kann gleichsam ein Fluid in die Kammer 11 eingezogen werden.

Das distale Ende 14 des Behältnisses 10 ist dazu mit einem Durchgangskanal 17, welcher von einer Spitze 12 umgeben ist, ausgebildet. Ein in die Kammer 11 eingefülltes Fluid (nicht abgebildet) kann dann bei einer entsprechenden Stopfenbewegung durch diesen Durchgangskanal 17 ausgetrieben werden, oder es kann auch bei einer umgekehrten Stopfenbewegung ein Fluid durch diesen Durchgangskanal 17 in die Kammer 11 eingezogen werden. Mit dem Bezugszeichen 18 ist die strichpunktiert dargestellte Mittenachse des Behältnisses 10 angegeben. Sie stellt gleichsam die Bezugsachse für die weiteren Komponenten dar.

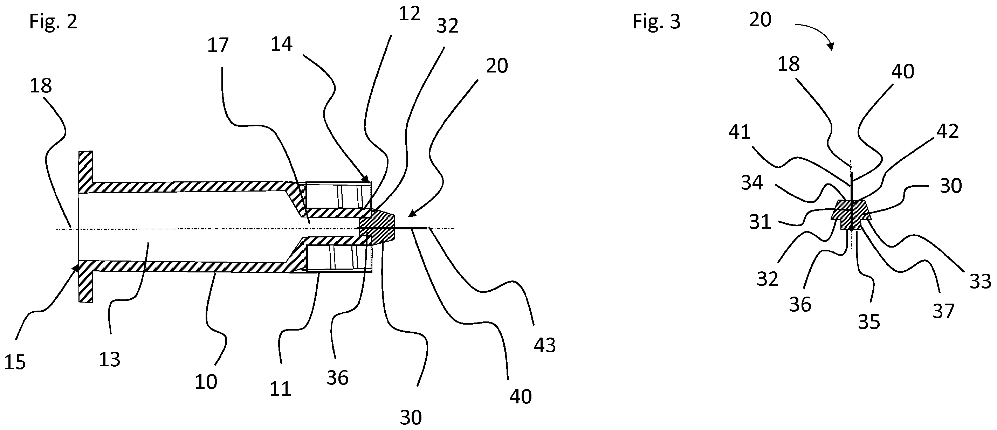

Eine wesentliche Komponente der Erfindung stellt die Kanülenbaugruppe 20 dar, welche exemplarisch in einem Ausführungsbeispiel in Fig. 3 schematisch in einer Schnittansicht dargestellt ist.

Die Fig. 2 zeigt ergänzend eine Schnittansicht eines Behältnis 10 mit einer aufgeschobenen Kanülenbaugruppe 20, umfassend ein Dichtelement 30 und eine Kanüle 40. Die Kanülenbaugruppe gemäß der in den Figuren 2 und 3 gezeigten Ausführungsform umfasst
ein zylindersymmetrisches Dichtelement 30 mit einem axialen Durchgang 31, und
eine Kanüle 40 mit einem durchgehenden Kanal 41,
wobei die Kanüle 40 koaxial zum Dichtelement 30 und zumindest abschnittsweise innerhalb des Durchgangs 31 des Dichtelements 30 angeordnet ist, und
wobei ein Abschnitt der Mantelfläche 42 der Kanüle 40 stoff- und/oder formschlüssig fest mit dem Dichtelement 30 verbunden ist und
wobei zumindest eine Stirnseite 33 des Dichtelements 30 einen den Durchgang 31 umgebenden,
kreisringförmigen Bereich umfasst, welcher eine stirnseitige Dichtfläche 32 in einer Ebene senkrecht zur Mittenachse 18 des Dichtelements 30 bildet.

Eine stoffschlüssige Verbindung meint hierbei wie bereits vorstehend ausgeführt, dass ein Zusammenhalt der Verbindungspartner durch atomare oder molekulare Kräfte geschaffen wird. Eine formschlüssige Verbindung meint hierbei, dass ein Ineinandergreifen von mindestens zwei Verbindungspartnern so erfolgt, dass diese eng aneinander anliegen, wobei durch dieses enge aneinander Anliegen auch eine fluiddichte Abdichtung bereitgestellt wird.

Der axiale Durchgang 31 des Dichtelements 30 dient der Aufnahme der Kanüle 40, also einer Hohlnadel oder einer Injektionsnadel mit einem durchgehenden Kanal 41, welche vorzugsweise im medizinischen Bereich eingesetzt werden kann. Die Verbindung zwischen Dichtelement 30 und Kanüle 40 ist dabei flüssigkeits- und/oder gasdicht im Sinne der Erfindung ausgeführt.

Die Erfindung macht auf besonders günstige Weise die Nutzung von Kanülen von 27 G bis 30 G, bevorzugt 31 G und 32 G und besonders bevorzugt 33 G und 34 G und darüber möglich. Dieses ist besonders günstig für eine patientenfreundliche Applikation eines pharmazeutischen Präparates.

Hierbei wird einem Patienten durch den sehr kleinen Durchmesser der erfindungsgemäß verwendbaren Kanüle sowohl bei einer Injektion als auch bei einer Entnahme von Körperflüssigkeiten nur noch ein stark vermindertes Schmerzempfinden zugemutet. Äußerst vorteilhaft wird hierdurch ein Injizieren oder Applizieren eines medizinischen Präparats in der Oralchirurgie insbesondere die Injektion eines Anästhetikums im Mundraum eines Patienten unterstützt, denn gerade der Mundraum ins regelmäßig durch ein erhöhtes Schmerzempfinden gekennzeichnet.

Aber auch das Injizieren oder Applizieren eines medizinischen Präparats in der Ophthalmologie, insbesondere zur Unterdrückung von Lidreflexen oder zur Weitstellung der Pupillen eines Patienten wird stark unterstützt, denn der erfindungsgemäß verwendbare geringe Kanülendurchmesser führt nur zu sehr kleinen Verletzungen oder Gewebeschädigungen, welche sich erfahrungsgemäß während des Heilungsprozesses sehr schnell wieder schließen.

Beim Injizieren oder Applizieren eines medizinischen Präparats in der der Nephrologie, insbesondere zur Applikation von Marker-Substanzen zur Verfolgung fluiddynamischer Prozesse können nun auch sehr kleine Gefäße erreicht werden und wird auch bei robotischen 3D-gesteuerten Anwendungen eine höhere Genauigkeit bei der Erfassung fluiddynamischer Prozesse erreicht.

Die im wesentlichen gleichen Vorteile treten auch auf beim Injizieren oder Applizieren eines medizinischen Präparats in der Nuklearmedizin zur Applikation radioaktiv emittierender Isotope, beispielsweise zur Tumorbehandlung, wobei dann Wirkstoffe des jeweiligen Präparats mit geringerer Schmerzverursachung und sowohl lokal als auch quantitativ genauer dosiert verabreicht werden können.

Als weniger unangenehm wird mit den erfindungsgemäßen Gegenständen auch das Injizieren oder Applizieren eines kosmetischen Präparats, wie beispielsweise von Hyaluronsäure oder eines Botox-basierten Präparates empfunden.

Vorteilhaft ist hierbei die Verwendung eines vorgefüllten Behälters, wie dieser vorliegend offenbart wird für eine sterile Lagerung eines pharmazeutischen oder kosmetischen Präparats für eine Lagerdauer von mehr als einem, bevorzugt mehr als zwei, besonders bevorzugt mehr als drei Jahren.

In dem gezeigten Ausführungsbeispiel weist die Kanüle lediglich beispielhaft eine Größe von 34 G auf.

Die Kanüle 40 ist in der montierten Position derart in dem Dichtelement 30 angeordnet, dass sie das Dichtelement 30 vollständig durchdringt. Dabei beträgt der Überstand auf der Seite mit der stirnseitigen Dichtfläche 32 in dem Ausführungsbeispiel etwa 0,2 mm, wobei ein Überstand in einem Bereich von 0,1 mm bis hin zu 3 mm, bevorzugt bis zu 2 mm von Vorteil ist, um zu verhindern, dass das Dichtelement den Eingang der Hohlnadel überdeckt.

Der Überstand auf der gegenüberliegenden Seite beträgt in dem Ausführungsbeispiel etwa 4 cm, wobei ein Überstand in einem Bereich von etwa 0,6 cm (2/8 Inch) bis hin zu etwa 5 cm (2 Inch) günstig ist, um den erforderlichen Freiraum der Kanüle 40 gegenüber dem Behältnis 10 im Gebrauch zu schaffen.

Das Dichtelement 30 ist aus einem elastomeren Material gefertigt und umfasst ein thermoplastisches Elastomer, insbesondere Butyl-, Chlorbutyl-, Brombutyl- oder Polyisoprenkautschuk. Dabei ist es für eine Nutzung in Kontakt mit einem pharmazeutischen Präparat zugelassen.

Um eine hinreichend breite Dichtfläche zwischen der Stirnseite der Spitze 12 und der stirnseitigen Dichtfläche 32 zur Verfügung stellen zu können, verfügt die stirnseitige Dichtfläche 32 über eine Ausdehnung in radialer Richtung von etwa 0,8 mm, wobei eine Ausdehnung von wenigstens 0,5 mm, bevorzugt von wenigstens 0,7 mm und besonders bevorzugt von wenigstens 1 mm als günstig angesehen wird.

Um die Dichtwirkung weiter zu verbessern und zusätzlich zu einer axialen Kompression auch eine radiale Kompression bei dem Abdichten zu erzielen, ist die Dichtfläche 32 in dem Ausführungsbeispiel mit einem zentrischen Vorsprung 36 ausgebildet. In dem Ausführungsbeispiel ist dieser Vorsprung 36 kegelstumpfförmig ausgebildet, was ein besonders einfaches Fügen durch eine Selbstjustage im Zuge des Verbindens von Kanülenbaugruppe 20 und Behältnis 10 ermöglicht und zudem eine gleichmäßige radiale Kompression sicherstellt.

Die radiale Kompression lässt sich, insbesondere bei Ausführungsformen mit einem kegelstumpfförmig ausgebildeten Vorsprung 36, durch die Steigung beziehungsweise den Winkel zwischen der Mittenachse des Dichtelements 18 und der Mantellinie des kegelstumpfförmigen Vorsprungs beeinflussen. Bevorzugt beträgt dieser wenigstens 1°, bevorzugt wenigstens 8° und besonders bevorzugt wenigstens 10° bis 30°.

Der zentrische Vorsprung 36 greift in montierter Position zumindest abschnittsweise in den Durchgangskanal 17 ein beziehungsweise ragt in diesen hinein. Auf diese Weise kann besonders günstig eine Erhöhung der Dichtwirkung erreicht werden zwischen der Öffnung des Durchgangskanals 17 und dem Dichtelement 30, wobei sich der weitere Vorteil ergibt, dass die Abdichtung zwischen Dichtelement 30 und Durchgangskanal 17 bereits in dem Durchgangskanal 17 selbst erfolgt, so dass kein Fluid in den Spalt zwischen den Stirnseiten von Spitze 17 und Dichtelement 30 gelangen kann.

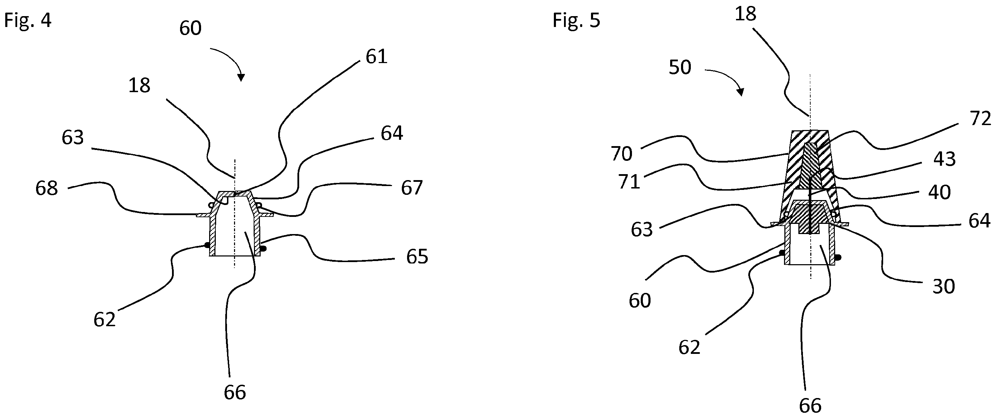

Die Kanülenbaugruppe 20 kann auf die Spitze 12 des Behältnisses 10 aufgesetzt werden. Das Haltern der Kanülbenbaugruppe 20 auf der Spitze 12 und das Erzeugen der erforderlichen Kompression in einer axialen und/oder radialen Richtung erfolgt mittels des erfindungsgemäßen Adapters 60, welcher in Fig. 4 in einer Schnittansicht in einem Ausführungsbeispiel gezeigt ist.

Adapter 60 und Kanülenbaugruppe 20 können gemeinsam das erfindungsgemäße Verschlusssystem 50 für Behältnisse 10 darstellen, um einen vorfüllbaren oder vorgefüllten Behälter 1 zu erzeugen. Ein Verschlusssystem 50 ist in Fig. 5 in einer Schnittansicht gezeigt ist, umfassend eine Kanülenbaugruppe nach dem Ausführungsbeispiel der Fig. 3 und einen Adapter nach dem Ausführungsbeispiel der Fig. 4. Das Verschlusssystem 50 der Fig. 5 umfasst ferner eine Verschlusskappe 70, welche auf den Adapter 60 aufgeschoben und gehaltert ist.

Der Adapter 60 dient dem Verbinden der Kanüle 40 mit dem Behältnis 10. Er ist als langgestreckter Hohlkörper ausgebildet mit einer axialen Durchgangsöffnung 61, einem proximalen Ende 65 und einem distalen Ende 64,
wobei der Innendurchmesser der Durchgangsöffnung 61 an dem proximalen Ende 65 erweitert ist und eine Aufnahme 66 zur Halterung des Dichtelements 30 bildet,
und wobei das proximale Ende 15 ferner ein Außengewinde 62 als Teil einer Luer-Lock-Verbindung umfasst, welches mit dem Innengewinde 11 des Behältnisses 10 verbunden ist oder verbunden werden kann. Als langgestreckt wird hierbei verstanden, dass der Adapter 60 zumindest entsprechend den nachfolgend zitierten Standards in Richtung der Mittellinie 18 die Länge eines Luer-Lock-Außengewindes oder eines auch als weiblich bezeichneten Luer-Lock-Anschlusses aufweist.

Das Außengewinde 62 des Adapters 60 ist als (weiblicher) Teil einer Luer-Lock-Verbindung ausgebildet und entspricht damit den entsprechenden Normen ISO 11040, ISO 80369-1 bzw. ISO 80369-7.

Die Aufnahme 66 ist geometrisch auf die Außengeometrie des aufzunehmenden Dichtelements 30 der Kanülenbaugruppe 20 abgestimmt und ermöglicht es somit, das Dichtelement 30 von der Stirnseite aus zumindest abschnittsweise in den Adapter 60 axial einschieben zu können bis zu einem Anschlag 63, der durch den Übergang der im Innendurchmesser erweiterten Durchgangsöffnung zu dem geringeren Innendurchmesser gebildet wird und der hierdurch ein weiteres Einschieben des Dichtelements 30 verhindert. In dem Ausführungsbeispiel ist die Aufnahme 66 in Übereinstimmung mit der Außenkontur des Dichtelements 30 nach innen konisch zulaufend ausgebildet. Dies ermöglicht es, das Dichtelement 30 mit geringer Kraft in die Aufnahme 66 axial einschieben zu können und in der Aufnahme 66 radial zu zentrieren.

Der Adapter 60 verfügt in dem Ausführungsbeispiel ferner über einen Ansatz 67, der es ermöglicht, die Verschlusskappe 70 auf das distale Ende 64 aufzustecken und in der vorgesehenen Position zu haltern. Die Verschlusskappe 70 ist dazu vorgesehen, in montierter Position den gegenüber dem Adapter 60 hervorstehenden Teil der Kanüle 40 zu schützen und die außenseitige Kanalöffnung 43 des Kanals 41 der Kanüle 40 im Sinne der Erfindung dicht, also flüssigkeits- und/oder gasdicht, zu verschließen.

Der Adapter 60 verfügt in dem Ausführungsbeispiel ferner über einen Montagering 68, der als Sechseck-Ring ausgeführt ist. Dieser unterstützt das Handling und erleichtert damit einem Benutzer die Montage an oder auf dem Behältnis 10.

Bei dem Ausführungsbeispiel der Fig. 5 ist die Kanülenbaugruppe 20 in den Adapter 60 bis zu der vorgesehenen Position eingeschoben. Die auf den Adapter 60 aufgesteckte und durch den Ansatz 67 gehalterte Verschlusskappe 70 verfügt in dem Ausführungsbeispiel über einen harten äußeren Bereich 71 und einen abdichtenden, elastomeren Innenbereich 72. Der gegenüber dem Adapter 60 hervorstehende Teil der Kanüle 40 ragt zumindest abschnittsweise in den elastomeren Innenbereich 72 der Verschlusskappe 70 hinein. Um eine sichere Abdichtung zu gewährleisten, sollte in montierter Position die Kanüle 40 wenigstens 2 mm, bevorzugt wenigstens 3 mm und besonders bevorzugt wenigstens 4 mm oder mehr in den elastomeren Innenbereich 72 hineinragen. In diesem Fall übt das elastomere Material 72 einen leichten Druck in axialer und/oder radialer Richtung auf die Kanalöffnung 43 der Kanüle 40 aus, wenn die Verschlusskappe 70 aufgesetzt ist, und verschließt diese dicht im Sinne der Erfindung.

Diese Abdichtung ermöglicht es, den Kanal 41 der Kanüle 40 gegenüber der Umgebung flüssigkeits- und/oder gasdicht abzudichten. Auf diese Weise kann auch die geforderte stabile und sterile Langzeitlagerfähigkeit gewährleistet werden.

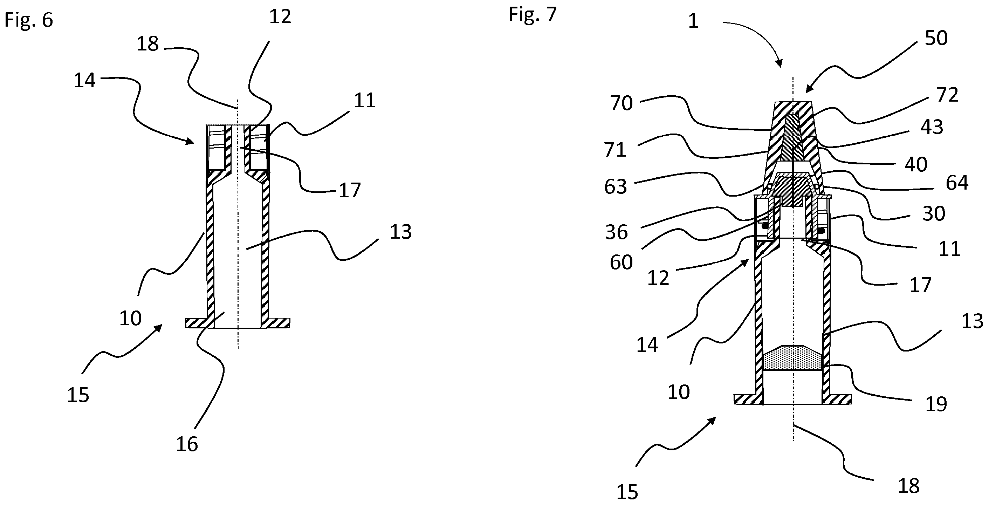

Fig. 6 zeigt ein erfindungsgemäß geeignetes Behältnis 10 in einer Schnittansicht am Beispiel einer Spritze. Das Behältnis 10 ist in diesem Beispiel aus einem transparentem Material gefertigt, im Beispiel aus dem thermoplastischen Kunststoff Cyclo-Olefin-Polymer (COP). Auch andere Materialien, etwa duroplastische Kunststoffe oder auch Glas, kommen als Material für das Behältnis 10 in Frage.

Geeignete thermoplastische Kunststoff umfassen ein Cyclo-Olefin-Copolymer (COC), Cyclo-Olefin-Polymer (COP), Acrylnitril-Butadien-Styrol (ABS), Polyamid (PA), Polylactat (PLA), Polymethylmethacrylat (PMMA), Polycarbonat (PC) oder Polyethylenterephtalat (PET).

Geeignete duroplastische Kunststoffe umfassen ein Celluloseacetat (CA) oder ein transparentes duroplastisches Harz oder bestehen aus diesem.

Geeignete Gläser umfassen ein Borosilikatglas, vorzugsweise mit einer Zusammensetzung (in Gew.-%):

| | |
|---|---|
| SiO₂ | 65% bis 82% |
| B₂O₃ | 5% bis 13% |
| ∑ Na₂O + K₂O | 4% bis 10% |
| Al₂O₃ | 2% bis 9% |
| Σ CaO + MgO | 0% bis 5% |

Fig. 7 schließlich zeigt in einer weiteren Schnittansicht einen vorfüllbaren Behälter 1 zum Lagern und/oder Applizieren eines Fluids, beispielsweise eines pharmazeutischen oder kosmetischen Präparates.

Der erfindungsgemäße vorfüllbare Behälter 1 umfasst ein Behältnis 10 am Beispiel eine Spritze wie in Fig. 2 oder 6 gezeigt, und ein montiertes Verschlusssystem 50, wie in Fig. 5 gezeigt, mit einer Kanülenbaugruppe 20 gemäß dem Ausführungsbeispiel in Fig. 3 und einem Adapter 60 gemäß dem Ausführungsbeispiel in Fig. 4.

Rein beispielhaft ist ein Stopfen 19 eingezeichnet, welcher die Öffnung 16 am proximale Ende 15 des Behältnisses verschließt. Ein Fluid, beispielsweise ein pharmazeutisches oder kosmetisches Präparat, kann in die Kammer 13 eingefüllt werden.

Das Verschlusssystem 50 und das Behältnis 60 sind mittels Luer-Lock-Verbindung miteinander verbunden. In montierter Position steht die Kanüle 40 in Fluidkontakt mit er Kammer 13 des Behältnisses. Der Adapter 60 ermöglicht es, eine Kanüle 40, vorzugsweise eine Kanüle mit einer Gauge-Größe von größer 30 G, bevorzugt von 31 G, 32 G, 33 G oder 34 G fest und dauerhaft dicht mit dem Behältnis 10 zu verbinden.

Durch das Verschlusssystem 50 wird in montierter Position eine Anpresskraft des Dichtelements 30 auf die Stirnseite der Spitze 12 von wenigstens 0,1 bis 30 N, bevorzugt 5 bis 20 N erzeugt.

Die Erfindung ermöglicht es auf besonders einfache Weise, ein Behältnis 10 mit einer zu verbinden und auf diese Weise den erfindungsgemäßen vorfüllbaren oder vorgefüllten Behälter 1 zur Verfügung zu stellen.

Bei einem erfindungsgemäß vorgefüllten Behälter 1 ist dabei die Kammer 13 zumindest zum Teil mit einem Fluid gefüllt. Das Fluid kann ein pharmazeutisches Präparat oder auch eine vorzugsweise flüssige, kosmetische Zubereitung umfassen.

Erfindungsgemäß kann somit auch ein vorgefüllter Behälter 1 mit einer Flüssigkeit, bevorzugt mit einer kosmetischen Zubereitung, zur Verfügung gestellt werden, welcher dadurch gekennzeichnet ist, dass die Kanüle 40 eine Gauge-Größe von größer 30 G, bevorzugt von 31 G, 32 G, 33 G oder 34 G aufweist. gefüllt ist, und bei welcher die Kanüle 40 eine Gauge-Größe von größer 30 G, bevorzugt von 31 G, 32 G, 33 G oder 34 G aufweist.

### Bezugszeichenliste:

- 1: vorfüllbarer oder vorgefüllter Behälter

- 10: Behältnis
- 11: Luer-Lock Innengewinde
- 12: Spitze
- 13: Kammer
- 14: distales Ende
- 15: proximales Ende
- 16: Öffnung
- 17: Durchgangskanal
- 18: Mittenachse
- 19: Stopfen

- 20: Kanülenbaugruppe

- 30: Dichtelement
- 31: Durchgang
- 32: stirnseitige Dichtfläche
- 33: Stirnseite
- 34: distales Ende
- 35: proximales Ende
- 36: Vorsprung
- 37: Mantellinie

- 40: Kanüle
- 41: Kanal
- 42: Mantelfläche
- 43: Kanülenöffnung

- 50: Verschlusssystem

- 60: Adapter
- 61: Durchgangsöffnung
- 62: Außengewinde
- 63: Anschlag
- 64: distales Ende
- 65: proximales Ende
- 66: Aufnahme
- 67: Ansatz
- 68: Montagering

- 70: Verschlusskappe
- 71: Außenbereich
- 72: elastomerer Innenbereich

## Patentansprüche

1. Vorfüllbarer oder vorgefüllter Behälter (1) zum Lagern und/oder Applizieren eines Fluids, insbesondere eines pharmazeutischen oder kosmetischen Präparates, umfassend
ein Behältnis (10),
mit
einer Kammer (13) zur Aufnahme
des Fluids
ferner umfassend
eine Kanülenbaugruppe (20),
wobei die Kanülenbaugruppe eine Kanüle (40) und
ein Dichtelement (30) aufweist, wobei die Kanüle (40) bündig mit dem Dichtelement (30) abschließt auf derjenigen Seite, welche in montierter Position zu der Kammer (13) hinweist, wobei zumindest eine Stirnseite (33) des Dichtelements (30), welche in montierter Position zu dem Behälter hinweist, einen einen Durchgang (31) umgebenden Bereich umfasst, welcher eine stirnseitige Dichtfläche (32) in einer Ebene senkrecht zur Mittenachse (18) des Dichtelements (30) bildet,
und
die Kanülenbaugruppe (20) an dem Behältnis (10) anbringbar ist,
sowie umfassend eine Verschlusskappe (70),
wobei eine außenseitige Kanalöffnung (43) der Kanüle (40) in einen abdichtenden elastomeren Innenbereich (72) der Verschlusskappe (70) hineinragt und dieser die Kanalöffnung (43) abdichtet,
wobei
durch eine radiale und/oder axiale Kompression des Dichtelements (30) sowie durch die Abdichtung der Kanalöffnung (43) mittels der Verschlusskappe (70) die Kammer (13) fluiddicht verschlossen ist.

2. Vorfüllbarer oder vorgefüllter Behälter (1) nach vorstehendem Anspruch, bei welchem
das Behältnis (10) einen Körper mit einem proximalen Ende (15) und einem distalen Ende (14) umfasst, und das distale Ende (14) einen axialen Durchgangskanal (17) mit einer den Durchgangskanal (17) umgebenden Spitze (12) aufweist, und wobei das distale Ende (14) ein Innengewinde (11) als Teil einer Luer-Lock-Verbindung umfasst, wobei das Dichtelement (30) der Kanülenbaugruppe (20) vorzugsweise ein zylindersymmetrisches Dichtelement (30) mit einem axialen Durchgang (31) und der Kanüle (40) umfasst, wobei die Kanüle (40) vorzugsweise koaxial zum Dichtelement (30) und zumindest abschnittsweise innerhalb des axialen Durchgangs (31) des Dichtelements (30) angeordnet ist, wobei ein Abschnitt der Mantelfläche (42) der Kanüle (40) vorzugsweise stoff- und/oder formschlüssig fest mit dem Dichtelement (30) verbunden ist und, ferner umfassend einen Adapter (60) zum Verbinden der Kanülenbaugruppe (20) mit der Spitze (12) des Behältnisses (10), wobei der Adapter (60) als ein insbesondere langgestreckter Hohlkörper ausgebildet ist, mit einer axialen Durchgangsöffnung (61), einem proximalen Ende (65) und einem distalen Ende (64), wobei der Innendurchmesser der Durchgangsöffnung (61) an dem proximalen Ende (65) erweitert ist und eine Aufnahme (66) zur Halterung des Dichtelements (30) bildet, wobei das proximale Ende (65) ferner ein Außengewinde (62) als Teil einer Luer-Lock-Verbindung umfasst, welches mit dem Innengewinde des Behältnisses (10) verbunden ist oder verbunden werden kann, wobei in montierter Position die Kanülenbaugruppe (20) in die Aufnahme (66) eingesetzt ist und den Durchgangskanal (17) abdichtet, wenn der Adapter (60) mit dem Behältnis (10) verbunden ist, und wobei in montierter Position die Kanüle (40) mit der Kammer (13) kommuniziert.

3. Behälter (1) nach einem der beiden vorstehenden Ansprüche, **dadurch gekennzeichnet, dass** das Behältnis (10) aus einem transparentem Material gefertigt ist, vorzugsweise aus Glas oder Kunststoff, vorzugsweise umfassend einen thermoplastischen oder duroplastischen Kunststoff.

4. Behälter (1) nach vorstehendem Anspruch, **dadurch gekennzeichnet, dass** der Behälter (1) aus thermoplastischen Kunststoff gefertigt ist und der thermoplastische Kunststoff ein Cyclo-Olefin-Copolymer (COC), Cyclo-Olefin-Polymer (COP), Acrylnitril-Butadien-Styrol (ABS), Polyamid (PA), Polylactat (PLA), Polymethylmethacrylat (PMMA), Polycarbonat (PC) oder Polyethylenterephtalat (PET) umfasst oder aus diesem besteht.

5. Behälter (1) nach vorstehendem Anspruch 3, **dadurch gekennzeichnet, dass** der Behälter (1) aus duroplastischen Kunststoff gefertigt ist und der duroplastische Kunststoff ein Celluloseacetat (CA) oder ein transparentes duroplastisches Harz umfasst oder aus diesem besteht.

6. Behälter (1) nach vorstehendem Anspruch 3, **dadurch gekennzeichnet, dass** der Behälter (1) aus Glas gefertigt ist und das Glas ein Borosilikatglas ist, vorzugsweise mit einer Zusammensetzung (in Gew.-%):
| | |
|---|---|
| SiO₂ | 65% bis 82% |
| B₂O₃ | 5% bis 13% |
| Σ Na₂O + K₂O | 4% bis 10% |
| Al₂O₃ | 2% bis 9% |
| Σ CaO + MgO | 0% bis 5% |

7. Behälter (1) nach einem der vorstehenden Ansprüche, **dadurch gekennzeichnet, dass** die Kanüle (40) eine Gauge-Größe von 27 G bis 30 G, bevorzugt von 31 G, 32 G, 33 G oder 34 G, aufweist.

8. Behälter (1) nach einem der vorstehenden Ansprüche, **dadurch gekennzeichnet, dass** die Kanüle (40) gegenüber dem Dichtelement (30) einen Überstand auf der Seite der stirnseitigen Dichtfläche (32) von 0,1 mm bis 3 mm, bevorzugt bis zu 2 mm aufweist, und/oder einen Überstand gegenüber dem Dichtelement (30) auf der gegenüberliegenden Seite in einem Bereich von etwa 0,6 cm (2/8 Inch) bis zu etwa 5 cm (2 Inch) aufweist.

9. Behälter (1) nach einem der vorstehenden Ansprüche, **dadurch gekennzeichnet, dass** die stirnseitige Dichtfläche (32) einen zentrischen, vorzugsweise kegelstumpfförmigen Vorsprung (36) umfasst, welcher den zentrischen Durchgang (31) umgibt, und welcher vorzugsweise zumindest abschnittsweise in den Durchgangskanal (17) eingreift oder in diesen hineinragt.

10. Behälter (1) nach dem vorstehenden Anspruch, **dadurch gekennzeichnet, dass** der Winkel zwischen der Mittenachse (18) und der Mantellinie (37) des kegelstumpfförmigen Vorsprungs (36) wenigstens 1°, bevorzugt wenigstens 8° und besonders bevorzugt wenigstens 10° bis 30° beträgt.

11. Behälter (1) nach einem der vorstehenden Ansprüche, **dadurch gekennzeichnet, dass** der Adapter (60) an seinem distalen Ende (64) einen Ansatz (67) zum Aufstecken und Haltern der Verschlusskappe (70) umfasst.

12. Behälter (1) nach einem der vorstehenden Ansprüche, mit einem Fluid oder mit einer Flüssigkeit, bevorzugt mit einer kosmetischen Zubereitung, **dadurch gekennzeichnet, dass** die Kanüle (40) eine Gauge-Größe von größer 30 G, bevorzugt von 31 G, 32 G, 33 G oder 34 G aufweist.

13. Behälter nach einem der vorstehenden Ansprüche, wobei der Behälter eine Spritze mit einem Spritzenkörper, eine Karpule oder eine Injektionsvorrichtung umfasst.

14. Behälter (1) nach einem der vorstehenden Ansprüche, wobei die Stirnseite (33) des Dichtelements (30), welche in montierter Position zu dem Behälter hinweist, einen einen Durchgang (31) umgebenden, kreisringförmigen Bereich umfasst.

15. Verwendung eines vorgefüllten Behälters nach einem der Ansprüche 1 bis 14 für eine sterile Lagerung eines pharmazeutischen oder kosmetischen Präparats für eine Lagerdauer von mehr als einem, bevorzugt mehr als zwei, besonders bevorzugt mehr als drei Jahren.

## Claims

1. A prefillable or prefilled container (1) for storing and/or applying a fluid, in particular a pharmaceutical or cosmetic preparation, comprising
a receptacle (10) with
a cavity (13) for holding the fluid;
furthermore comprising
a cannula assembly (20);
wherein said cannula assembly comprises a cannula (40) and
a sealing element (30), wherein the cannula (40) is flush with the sealing element (30) on the side facing the cavity (13) in the assembled position, wherein at least one end face (33) of the sealing element (30), which faces the receptacle in the assembled position, comprises a zone surrounding a bore (31), which defines an end-side sealing surface (32) in a plane perpendicular to the central axis (18) of the sealing element (30); and
wherein said cannula assembly (20) can be attached to the receptacle (10);
and comprising a closure cap (70);
wherein an external channel opening (43) of the cannula (40) projects into a sealing elastomeric interior (72) of the closure cap (70), which seals the channel opening (43);
wherein the cavity (13) is closed in a fluid-tight manner by virtue of a radial and/or axial compression of the sealing element (30) and by virtue of the sealing of the channel opening (43) by the closure cap (70).

2. The prefillable or prefilled container (1) according to the preceding claim, wherein the receptacle (10) comprises a body having a proximal end (15) and a distal end (14), and the distal end (14) having an axial through-passage (17) with a tip (12) surrounding the through-passage (17), and wherein the distal end (14) comprises an internal thread (11) as part of a Luer-lock connection, wherein the sealing element (30) of the cannula assembly (20) preferably comprises a cylindrically symmetrical sealing element (30) having an axial bore (31) and the cannula (40), wherein the cannula (40) is preferably arranged coaxially relative to the sealing element (30) and at least partially inside the axial bore (31) of the sealing element (30), wherein a section of the lateral surface (42) of the cannula (40) is preferably firmly coupled to the sealing element (30) by a material bond or in a form-fitting manner; and further comprising an adapter (60) for connecting the cannula assembly (20) to the tip (12) of the receptacle (10), wherein the adapter (60) is in particular designed as an elongated hollow body having an axial through-opening (61), a proximal end (65) and a distal end (64), wherein the inner diameter of the through-opening (61) is widened at the proximal end (65) to form a seat (66) for holding the sealing element (30), with the proximal end (65) further comprising an external thread (62) as part of a Luer-lock connection, which is or can be coupled to the internal thread of the receptacle (10), wherein in the assembled position the cannula assembly (20) is inserted in the seat (66) and seals the through-passage (17) when the adapter (60) is connected to the receptacle (10), and wherein in the assembled position the cannula (40) is in communication with the cavity (13).

3. The container (1) according to any one of the two preceding claims, **characterized in that** the receptacle (10) is made of a transparent material, preferably of glass or plastics material, preferably comprising a thermoplastic or thermosetting plastics material.

4. The container (1) according to the preceding claim, **characterized in that** the container (1) is made of a thermoplastic material and the thermoplastic material comprises or is made of a cyclic olefin copolymer (COC), cyclic olefin polymer (COP), acrylonitrile-butadiene-styrene (ABS), polyamide (PA), polylactic acid (PLA), polymethyl methacrylate (PMMA), polycarbonate (PC) or polyethylene terephthalate (PET).

5. The container (1) according to the preceding claim 3, **characterized in that** the container (1) is made of a thermosetting plastics material and the thermosetting plastics material comprises or is made of a cellulose acetate (CA) or a transparent thermosetting resin.

6. The container (1) according to the preceding claim 3, **characterized in that** the container (1) is made of glass and the glass is a borosilicate glass, preferably with a composition (in wt%) of:
| | |
|---|---|
| SiO₂ | 65 % to 82 %, |
| B₂O₃ | 5 % to 13 %, |
| Σ Na₂O + K₂O | 4 % to 10 %, |
| Al₂O₃ | 2 % to 9 %, |
| Σ CaO + MgO | 0 % to 5 %. |

7. The container (1) according to any one of the preceding claims, **characterized in that** the cannula (40) has a gauge size of 27G to 30G, preferably 31G, 32G, 33G or 34G.

8. The container (1) according to any one of the preceding claims, **characterized in that**, on the side of the end-side sealing surface (32), the cannula (40) projects from the sealing element (30) by 0.1 mm to 3 mm, preferably up to 2 mm, and/or projects from the sealing element (30) on the opposite side by approximately 0.6 cm (2/8 inch) to approximately 5 cm (2 inches).

9. The container (1) according to any one of the preceding claims, **characterized in that** the end-side sealing surface (32) comprises a central, preferably frustoconical projection (36) surrounding the central bore (31), and which preferably engages or projects into the through-passage (17), at least sections thereof.

10. The container (1) according to the preceding claim, **characterized in that** the angle between the central axis (18) and the generatrix (37) of the frustoconical projection (36) is at least 1°, preferably at least 8°, and most preferably at least 10° to 30°.

11. The container (1) according to any one of the preceding claims, **characterized in that** the adapter (60), at its distal end (64), comprises a lug (67) for fitting and retaining the closure cap (70).

12. The container (1) according to any one of the preceding claims, including a fluid or a liquid, preferably a cosmetic preparation, **characterized in that** the cannula (40) has a gauge size of greater than 30G, preferably 31G, 32G, 33G or 34G.

13. The container according to any one of the preceding claims, wherein the container comprises a syringe with a syringe body, a cartridge or an injection device.

14. The container (1) according to any one of the preceding claims, wherein the end face (33) of the sealing element (30) facing the receptacle in the assembled position comprises an annular zone surrounding a bore (31).

15. Use of a prefilled container according to any one of claims 1 to 14 for sterile storage of a pharmaceutical or cosmetic preparation over a storage period of more than one, preferably more than two, most preferably more than three years.

## Revendications

1. Contenant (1) prérempli ou pouvant être prérempli pour stocker et/ou appliquer un fluide, en particulier une préparation pharmaceutique ou cosmétique, comprenant un récipient (10),
avec
une chambre (13) pour recevoir
le fluide
comprenant en outre
un ensemble canule (20),
dans lequel l'ensemble canule présente une canule (40) et
un élément d'étanchéité (30), dans lequel la canule (40) se termine à fleur de l'élément d'étanchéité (30) sur la face qui, en position montée, est orientée vers la chambre (13), dans lequel au moins une face frontale (33) de l'élément d'étanchéité (30), qui, en position montée, est orientée vers le contenant, comprend une zone entourant un passage (31), laquelle forme une surface d'étanchéité frontale (32) dans un plan perpendiculaire à l'axe central (18) de l'élément d'étanchéité (30),
et
l'ensemble canule (20) peut être appliqué sur le récipient (10),
et comprenant un capuchon de fermeture (70),
dans lequel une ouverture de canal (43) côté extérieur de la canule (40) pénètre dans une zone interne élastomère d'étanchéité (72) du capuchon de fermeture (70) et celle-ci étanchéifie l'ouverture de canal (43),
dans lequel
la chambre (13) est fermée de manière étanche au fluide par une compression radiale et/ou axiale de l'élément d'étanchéité (30) ainsi que par l'étanchéification de l'ouverture de canal (43) à l'aide du capuchon de fermeture (70).

2. Contenant (1) prérempli ou pouvant être prérempli selon la revendication précédente, pour lequel
le récipient (10) comprend un corps avec une extrémité proximale (15) et une extrémité distale (14), et l'extrémité distale (14) présente un canal de passage (17) axial avec une pointe (12) entourant le canal de passage (17), et dans lequel l'extrémité distale (14) comprend un filetage intérieur (11) faisant partie d'un raccord Luer-Lock, dans lequel l'élément d'étanchéité (30) de l'ensemble canule (20) comprend de préférence un élément d'étanchéité (30) cylindriquement symétrique avec un passage axial (31) et la canule (40), dans lequel la canule (40) est disposée de préférence coaxialement à l'élément d'étanchéité (30) et au moins sur certaines sections à l'intérieur du passage axial (31) de l'élément d'étanchéité (30), dans lequel une section de la surface d'enveloppe (42) de la canule (40) est reliée fixement à l'élément d'étanchéité (30) de préférence par liaison de matière et/ou coopération de formes et comprenant en outre un adaptateur (60) pour relier l'ensemble canule (20) à la pointe (12) du récipient (10), dans lequel l'adaptateur (60) est réalisé comme un corps creux en particulier allongé, avec une ouverture de passage (61) axiale, une extrémité proximale (65) et une extrémité distale (64), dans lequel le diamètre intérieur de l'ouverture de passage (61) est élargi à l'extrémité proximale (65) et forme un logement (66) pour retenir l'élément d'étanchéité (30), dans lequel l'extrémité proximale (65) comprend en outre un filetage extérieur (62) faisant partie d'un raccord Luer-Lock, lequel est relié ou peut être relié au filetage intérieur du récipient (10), dans lequel, en position montée, l'ensemble canule (20) est inséré dans le logement (66) et étanchéifie le canal de passage (17) lorsque l'adaptateur (60) est relié au récipient (10), et dans lequel, en position montée, la canule (40) communique avec la chambre (13).

3. Contenant (1) selon l'une quelconque des deux revendications précédentes, **caractérisé en ce que** le récipient (10) est fabriqué à partir d'un matériau transparent, de préférence en verre ou en plastique, comprenant de préférence un plastique thermoplastique ou thermodurcissable.

4. Contenant (1) selon la revendication précédente, **caractérisé en ce que** le contenant (1) est fabriqué en plastique thermoplastique et le plastique thermoplastique comprend un copolymère de cyclooléfine (COC), un polymère de cyclooléfine (COP), l'acrylonitrile butadiène styrène (ABS), le polyamide (PA), le polylactate (PLA), le polyméthacrylate de méthyle (PMMA), le polycarbonate (PC) ou le polyéthylène téréphtalate (PET) ou est constitué de celui-ci.

5. Contenant (1) selon la revendication 3 précédente, **caractérisé en ce que** le contenant (1) est fabriqué en plastique thermodurcissable et le plastique thermodurcissable comprend un acétate de cellulose (CA) ou une résine thermodurcissable transparente ou est constitué de celui(celle)-ci.

6. Contenant (1) selon la revendication 3 précédente, **caractérisé en ce que** le contenant (1) est fabriqué en verre et le verre est un verre borosilicaté, de préférence avec une composition (en % en poids) :
| | |
|---|---|
| SiO₂ | 65 % à 82 % |
| B₂O₃ | 5 % à 13 % |
| Σ Na₂O + K₂O | 4 % à 10 % |
| Al₂O₃ | 2 % à 9 % |
| Σ CaO + MgO | 0 % à 5 % |

7. Contenant (1) selon l'une quelconque des revendications précédentes, **caractérisé en ce que** la canule (40) présente une taille de calibre de 27 G à 30 G, de préférence de 31 G, 32 G, 33 G ou 34 G.

8. Contenant (1) selon l'une quelconque des revendications précédentes, **caractérisé en ce que** la canule (40) présente par rapport à l'élément d'étanchéité (30) un dépassement sur le côté de la surface d'étanchéité frontale (32) de 0,1 mm à 3 mm, de préférence jusqu'à 2 mm, et/ou un dépassement par rapport à l'élément d'étanchéité (30) sur le côté opposé dans une plage d'environ 0,6 cm (2/8 pouces) à environ 5 cm (2 pouces).

9. Contenant (1) selon l'une quelconque des revendications précédentes, **caractérisé en ce que** la surface d'étanchéité frontale (32) comprend une saillie (36) centrale, de préférence tronconique, laquelle entoure le passage central (31), et laquelle s'insère de préférence au moins sur certaines sections dans le canal de passage (17) ou pénètre dans celui-ci.

10. Contenant (1) selon la revendication précédente, **caractérisé en ce que** l'angle entre l'axe central (18) et la génératrice (37) de la saillie tronconique (36) est d'au moins 1°, de préférence d'au moins 8° et de manière particulièrement préférée d'au moins 10° à 30°.

11. Contenant (1) selon l'une quelconque des revendications précédentes, **caractérisé en ce que** l'adaptateur (60) comprend à son extrémité distale (64) une embase (67) pour fixer et retenir le capuchon de fermeture (70).

12. Contenant (1) selon l'une quelconque des revendications précédentes, avec un fluide ou avec un liquide, de préférence avec une préparation cosmétique, **caractérisé en ce que** la canule (40) présente une taille de calibre supérieure à 30 G, de préférence de 31 G, 32 G, 33 G ou 34 G.

13. Contenant selon l'une quelconque des revendications précédentes,
dans lequel le contenant comprend une seringue avec un corps de seringue, une cartouche ou un
dispositif d'injection.

14. Contenant (1) selon l'une quelconque des revendications précédentes, dans lequel la face frontale (33) de l'élément d'étanchéité (30), laquelle est orientée vers le contenant en position montée, comprend une zone circulaire entourant un passage (31).

15. Utilisation d'un contenant prérempli selon l'une quelconque des revendications 1 à 14 pour un stockage stérile d'une préparation pharmaceutique ou cosmétique pendant une durée de plus d'un, de préférence plus de deux, et de manière particulièrement préférée plus de trois ans.
